# EUROPEAN PATENT SPECIFICATION

(11) **EP 3 507 380 B1**
(45) Date of publication and mention of the grant of the patent: **22.09.2021**
(21) Application number: 17762088.7
(22) Date of filing: 31.08.2017
(51) Int. Cl.: C12Q 1/6883

(54) **GENE PANEL SPECIFIC FOR PULMONARY HYPERTENSION AND ITS USES**
FÜR PULMONALE ARTERIELLE HYPERTONIE UND/ODER CHRONISCHE THROMBOEMBOLISCHE PULMONALE HYPERTONIE SPEZIFISCHES GEN-PANELUND DESSEN VERWENDUNGEN
PANNEAU DE GÈNES SPÉCIFIQUE POUR L'HYPERTENSION ARTÉRIELLE PULMONAIRE ET/OU L'HYPERTENSION PULMONAIRE THROMBO-EMBOLIQUE CHRONIQUE ET SES UTILISATIONS

(30) Priority: 02.09.2016 EP 16187084
(43) Date of publication of application: 10.07.2019
(73) Proprietor: Ruprecht-Karls-Universität Heidelberg, 69117 Heidelberg (DE); Thoraxklinik-Heidelberg gGmbH, 69126 Heidelberg (DE)
(72) Inventor: GRÜNIG, Ekkehard, 69198 Schriesheim (DE); HINDERHOFER, Katrin, 69118 Heidelberg (DE); EICHSTAEDT, Christina A., 69120 Heidelberg (DE)
(74) Representative: Grahn, Sibylla Maria
(86) International application number: PCT/EP2017/071886
(87) International publication number: WO 2018/041959

(56) References cited:
- Anonymous: "Pulmonary Arterial Hypertension - PAH", , 1 August 2016 (2016-08-01), XP055323855, Retrieved from the Internet: URL:https://arupconsult.com/content/pulmon ary-arterial-hypertension/?tab=tab_item-0 [retrieved on 2016-11-29] & Anonymous: "Pulmonary Arterial Hypertension (PAH) Sequencing, Multigene ", , 29 November 2016 (2016-11-29), XP055323834, Retrieved from the Internet: URL:http://ltd.aruplab.com/Tests/Pub/20093 50 [retrieved on 2016-11-29]
- AUSTIN ED; MA L; LEDUC C; BERMAN ROSENZWEIG E; BORCZUK A; PHILLIPS JA, 3RD ET AL.: "Whole exome sequencing to identify a novel gene (caveolin-1) associated with human pulmonary arterial hypertension", CIRC CARDIOVASC GENET, vol. 5, no. 3, 2012, pages 336-43, XP055323389, cited in the application
- DE JESUS PEREZ VA; YUAN K; LYUKSYUTOVA MA; DEWEY F; ORCHOLSKI ME; SHUFFLE EM ET AL.: "Whole-Exome Sequencing Reveals TopBPl as a Novel Gene in Idiopathic Pulmonary Arterial Hypertension", AM J RESPIR CRIT CARE MED., vol. 189, no. 10, 2014, pages 1260-72, XP055323405, cited in the application
- CHIDA A; SHINTANI M; NALCAYAMA T; FURUTANI Y; HAYAMA E; INAI K ET AL.: "Missense mutations of the BMPR1B (ALK6) gene in childhood idiopathic pulmonary arterial hypertension", CIRC J., vol. 76, no. 6, 2012, pages 1501-8, XP055323399, cited in the application
- NASIM MT; GHOURI A; PATEL B; JAMES V; RUDARAKANCHANA N; MORRELL NW ET AL.: "Stoichiometric imbalance in the receptor complex contributes to dysfunctional BMPR-II mediated signalling in pulmonary arterial hypertension", HUM MOL GENET, vol. 17, no. 11, 2008, pages 1683-94, XP055323960, cited in the application
- KWAPISZEWSKA G; RODRIGUEZ VIALES R; EHLKEN N; EICHSTAEDT C; RIEMKASTEN G; GRUNIG G ET AL.: "Epigenetik und Genetik der pulmonal arteriellen Hypertonie - neue Erkenntnisse der letzten Jahre", DTSCH MED WOCHENSCHR., vol. 139, no. 04, 2014, pages 111-5, XP055323963, cited in the application
- C. V. REMILLARD ET AL: "Function of Kv1.5 channels and genetic variations of KCNA5 in patients with idiopathic pulmonary arterial hypertension", AMERICAN JOURNAL OF PHYSIOLOGY. CELL PHYSIOLOGY., vol. 292, no. 5, 13 December 2006 (2006-12-13), pages C1837-C1853, XP055323968, US ISSN: 0363-6143, DOI: 10.1152/ajpcell.00405.2006 cited in the application
- PIAO C; ZHU Y; ZHANG C; XI X; LIU X; ZHENG S; LI X; GUO J; JIA L; NAKANISHI T: "Identification of multiple ACVRL1 mutations in patients with pulmonary arterial hypertension by targeted exome capture", CLIN SCI (LOND, vol. 130, no. 17, 28 June 2016 (2016-06-28), pages 1559-69, XP009192626, cited in the application

## Description

The present invention relates to a gene panel of at least 14 genes or the combination of said at least 14 genes for use in a method of diagnosis of a genetic disposition to pulmonary arterial hypertension (PAH) and/or chronic thromboembolic pulmonary hypertension (CTEPH). The present invention relates to using said gene panel or combination of genes in the *in vitro* or *ex vivo* diagnosis of a genetic disposition to pulmonary arterial hypertension (PAH) and/or chronic thromboembolic pulmonary hypertension (CTEPH). The present invention relates to a method and a kit for the diagnosis of a genetic disposition to pulmonary arterial hypertension (PAH) and/or chronic thromboembolic pulmonary hypertension (CTEPH). The present invention further relates to said gene panel, method and kit with respect to a genetic disposition to pulmonary veno-occlusive disease (PVOD) and/or hereditary haemorrhagic telangiectasia (HHT).

### BACKGROUND OF THE INVENTION

Pulmonary arterial hypertension (PAH) is characterised by plexiform lesions of the small pulmonary arteries leading to an increase in pulmonary vascular resistance and right heart failure (Galiè *et al.,* 2016; Grünig *et al.,* 2011). The recent decade witnessed the discovery of hereditary predisposition to PAH. In 2000, mutations in the bone morphogenic protein receptor type 2 (*BMPR2*) gene, a member of the transforming growth factor-beta (TGF-β) super family, have been identified in families with autosomal dominantly inherited PAH (hereditary PAH, HPAH) (see e.g. Deng *et al.,* 2000; Grünig *et al.,* 2000; Lane *et al.,* 2000). Defects of the *BMPR2* gene have been detected to be the major heritable cause for development of PAH (Machado *et al.,* 2015; Soubrier *et al.,* 2013) albeit having an incomplete penetrance of about 20-40% (Larkin *et al.,* 2012; Sztrymf *et al.,* 2007) in single families up to 50% (Newman *et al.,* 2001; Frydman *et al.,* 2012). In approximately 85% of familial PAH and 14-35% of sporadic idiopathic PAH (IPAH) cases, mutations of *BMPR2* gene were identified (Soubrier *et al.,* 2013; Pfarr *et al.,* 2011; Kabata *et al.,* 2013; Girerd *et al.,* 2016). In PAH patients with hereditary haemorrhagic telangiectasia (HHT) mutations in the activin receptor-like kinase 1 (*ALK1* or *ACVRL1*) (Trembath *et al.,* 2001) and endoglin (*ENG*) (Chaouat *et al.,* 2004; Harrison *et al.,* 2003) gene were documented. Therefore, diagnostic genetic testing within the last decade has concentrated on these 3 genes and is usually performed by direct Sanger sequencing in a sequential manner, terminating the sequencing as soon as a mutation has been identified in one of these genes. However, within the last years further genes belonging to the BMPR2/Alkl-signalling pathway have been detected by whole exome next generation sequencing (NGS) to be involved in the pathogenesis of PAH and/or pulmonary veno-occlusive disease (PVOD) and/or hereditary haemorrhagic telangiectasia (HHT). HHT often lead to pulmonary arterial hypertension which drastically worsens patients' survival (Lyle *et al.,* 2016). In hereditary PVOD cases 100% of hereditary patients carried two mutations in the gene *EIF2AK4* (Girerd *et al.,* 2016). The ERS/ESC guidelines thus suggest a mutation analysis to be preferred to a biopsy to ascertain the diagnosis of PVOD (Galiè *et al.,* 2016).

Recently mutations and large deletions were also discovered in the three major genes *BMPR2, ENG* and *ALK1* in patients with chronic thromboembolic pulmonary hypertension (CTEPH) (Feng *et al.,* 2014, Xi *et al.,* 2016). Therefore, it is likely that genetic defects also play a role in the manifestation of CTEPH.

The availability of genetic testing including genetic counselling has become more and more important for patient care and for family members of PAH patients. Therefore, genetic testing and counselling has been addressed and recommended in the new European Respiratory Society/ European Society of Cardiology (ERS/ESC)-guidelines for selected PAH patients and their family member (Galiè *et al.,* 2016). The most common molecular approach is genetic testing by Sanger technique of only 3 genes. This might no longer be appropriate, since today at least 14 PAH/CTEPH/PVOD/HHT genes are known and the Sanger technique assessing one gene at a time is a very time consuming and expensive method (at least 11 h analysis time -in reality 1-2 weeks turnaround time for the three major genes). Furthermore, there is growing evidence that in some patients at least 2 gene defects are necessary for disease manifestation (second hit hypothesis) (Eichstaedt *et al.,* 2016; Rodriguez Viales *et al.,* 2015; Wang *et al.,* 2014).

Pulmonary Arterial Hypertension - PAH. Arup Consult®. (https://arupconsult.com/content/ pulmonary-arterial-hypertension?tab=tab_item-0) discloses a method and kit for diagnosing PAH, wherein mutations in the six complete genes ACVLR1, BMPR2, CAV1, ENG, EIF2AK4, and KCNK3 are detected by performing parallel sequencing.

There is a need in the art for improved means and methods for diagnosing PAH/CTEPH and screening of PAH/CTEPH, including PVOD and/or HHT.

### SUMMARY OF THE INVENTION

The invention is disclosed in the appended claims. According to the present disclosure this object is solved by providing a gene panel or combination of genes comprising at least the genes with the nucleotide sequence of SEQ ID Nos. 1 to 14 for use in a method of diagnosis of a genetic disposition to pulmonary arterial hypertension (PAH) and/or chronic thromboembolic pulmonary hypertension (CTEPH).

According to the present disclosure this object is solved by using a gene panel or combination of genes comprising at least the genes with the nucleotide sequence of SEQ ID Nos. 1 to 14 in the *in vitro* or *ex vivo* diagnosis of a genetic disposition to pulmonary arterial hypertension (PAH) and/or chronic thromboembolic pulmonary hypertension (CTEPH).

According to the present disclosure this object is solved by a method for the diagnosis of genetic defects in pulmonary arterial hypertension (PAH) and/or chronic thromboembolic pulmonary hypertension (CTEPH), comprising the following steps:
(a) providing at least one sample,
   preferably at least two samples,
(b) extracting the genomic DNA from said sample(s),
(c) determining whether at least one mutation in terms of one or more altered base pairs, smaller and larger deletions or insertions in the genes with the nucleotide sequence of SEQ ID Nos. 1 to 14 is present by using next generation sequencing, wherein the presence of said at least one such mutation is a genetic confirmation of existing PAH or indicative for the increased probability for PAH/CTEPH to manifest in not yet affected relatives of PAH/CTEPH patients, and
(d) optionally, determining whether detected mutations/gene variants have pathological consequences either predicted with *in silico* prediction programmes and/or functional studies
wherein the sample(s) is/are patient sample(s) or sample(s) from a relative of a PAH patient.

According to the present disclosure this object is solved by providing a kit for the diagnosis of genetic defects in pulmonary arterial hypertension (PAH) and/or chronic thromboembolic pulmonary hypertension (CTEPH), comprising
(i) means for carrying out next generation sequencing and detecting at least one mutation in terms of one or more altered base pairs, smaller and larger deletions or insertions in the genes with the nucleotide sequence of SEQ ID Nos. 1 to 14,
(ii) optionally, means for processing genomic DNA,
(iii) optionally, means for carrying out an additional sequencing, preferably Sanger sequencing, or an analysis technique, preferably multiplex ligation-dependent probe amplification or quantitative polymerase chain reaction.

### DESCRIPTION OF THE PREFERRED EMBODIMENTS OF THE INVENTION

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "1 to 96" or "2 to 96" should be interpreted to include not only the explicitly recited values of 1 to 96, but also include individual values and sub-ranges within the indicated range. Thus, included in this numerical range are individual values such as 1, 2, 3, 4, 5 .... 91, 92, 93, 94, 95, 96 and sub-ranges such as from 2 to 10, 15 to 50, 1 to 90, etc. This same principle applies to ranges reciting only one numerical value, such as "at least 2". Furthermore, such an interpretation should apply regardless of the breadth of the range or the characteristics being described.

### Use of the PAH-specific panel

In this disclosure we disclose the development of a PAH/CTEPH-specific gene diagnostic panel using NGS and including 14 known PAH and further candidate genes. The new procedure is widely available, easy to perform and will allow assessing up to 50 genes and up to 96 patients at once in a short time at reduced costs. Furthermore, it is possible to assess sensitivity and specificity of this new panel in a cohort of patients/family members using additional Sanger sequencing.

Preferably, the use comprises the *in vitro* or *ex vivo* diagnosis of a genetic disposition to pulmonary veno-occlusive disease (PVOD) and/or hereditary haemorrhagic telangiectasia (HHT).

The terms "PAH/CTEPH" or "PAH/CTEPH/PVOD/HHT", such as when referring to gene(s) and/or patient(s), are used interchangeably throughout the specification.

As discussed above, the present disclosure provides a gene panel or combination of genes comprising at least the genes with the nucleotide sequence of SEQ ID Nos. 1 to 14 for use in a method of diagnosis of pulmonary arterial hypertension (PAH) and/or chronic thromboembolic pulmonary hypertension (CTEPH), in particular the diagnosis of a genetic predisposition to PAH and/or CTEPH.

As discussed above, the present disclosure provides the use of a gene panel or combination of genes comprising at least the genes with the nucleotide sequence of SEQ ID Nos. 1 to 14 in the *in vitro* or *ex vivo* diagnosis of pulmonary arterial hypertension (PAH) and/or chronic thromboembolic pulmonary hypertension (CTEPH), in particular the diagnosis of a genetic predisposition to PAH and/or CTEPH.

Preferably, the present disclosure provides the use of the gene panel or combination of genes in the *in vitro* or *ex vivo* diagnosis of pulmonary arterial hypertension (PAH), chronic thromboembolic pulmonary hypertension (CTEPH), pulmonary veno-occlusive disease (PVOD) and/or hereditary haemorrhagic telangiectasia (HHT), in particular the diagnosis of a genetic predisposition to PAH and/or CTEPH and/or PVOD and/or HHT.

In a preferred embodiment, the gene panel or combination of genes of the present invention is used which comprises the at least 14 genes with the nucleotide sequence of SEQ ID Nos. 1 to 14 and up to the 50 genes, wherein said 50 genes comprise the genes with the nucleotide sequence of SEQ ID Nos. 1 to 43.

In a preferred embodiment, the gene panel or combination of genes comprises the at least the genes with the nucleotide sequence of SEQ ID Nos. 1 to 14 and 42.

Preferably a range of at least 14 genes and up to 50 genes are used. In one embodiment 15 genes are used, in one embodiment 36 genes are used, in one embodiment 43 genes are used.

In a preferred embodiment, the gene panel or combination of genes is used which comprises at least the genes with the nucleotide sequence of SEQ ID Nos. 1 to 14 and 42, more preferably comprises the genes with the nucleotide sequence of SEQ ID Nos. 1 to 36, more preferably comprises the genes with the nucleotide sequence of SEQ ID Nos. 1 to 43.

The term "gene panel" or "combination of genes" or "gene set" or "set of genes" refers to the genes included on the panel which are either genes identified as mutated in PAH, CTEPH, PVOD and/or HHT patients or such genes which have been associated with PH in animal models and might contribute to disease manifestation in case of a genetic defect.

The inventors have found that the following set of 14 genes allows for the diagnosis of genetic defects of PAH and partly CTEPH, PVOD and/or HHT:

| **Gene** | **Ensemble Gene ID** | **SEQ ID NO.** |
|---|---|---|
| ***ACVLR1 (ALK1)*** | ENSG00000139567 | 1 |
| ***BMPR1B*** | ENSG00000138696 | 2 |
| ***BMPR2 (*+ *promoter)*** | ENSG00000204217 | 3 |
| ***CAV1*** | ENSG00000105974 | 4 |
| ***EIF2AK4*** | ENSG00000128829 | 5 |
| ***ENG*** | ENSG00000106991 | 6 |
| ***GDF2*** | ENSG00000263761 | 7 |
| ***KCNA5*** | ENSG00000130037 | 8 |
| ***KCNK3*** | ENSG00000171303 | 9 |
| ***KLF2*** | ENSG00000127528 | 10 |
| ***SMAD1*** | ENSG00000170365 | 11 |
| ***SMAD4*** | ENSG00000141646 | 12 |
| ***SMAD9*** | ENSG00000120693 | 13 |
| ***TOPBP1*** | ENSG00000163781 | 14 |

Mutations in these 14 genes or "core genes" have been shown to lead to PAH manifestation and partly to CTEPH, PVOD and/or HHT manifestation.

The genes *ACVRL1, BMRP2, ENG* (SEQ ID NOs. 1, 3, 6) have been found to be mutated in CTEPH.

The gene *EIF2AK4* (SEQ ID NO 5) has been shown to be mutated in PVOD.

The genes *ACVRL1, BMPR2, ENG, GDF2, SMAD4* (SEQ ID NOs. 1, 3, 6, 7, 12) have been shown to be mutated in HHT.

The inventors have found that the following set of 15 genes further allows for the diagnosis of genetic defects of PAH and partly CTEPH, PVOD and/or HHT:

| **Gene** | **Ensemble Gene ID** | **SEQ ID NO.** |
|---|---|---|
| ***ACVLR1 (ALK1)*** | ENSG00000139567 | 1 |
| ***BMPR1B*** | ENSG00000138696 | 2 |
| ***BMPR2 (*+ *promoter)*** | ENSG00000204217 | 3 |
| ***CAV1*** | ENSG00000105974 | 4 |
| ***EIF2AK4*** | ENSG00000128829 | 5 |
| ***ENG*** | ENSG00000106991 | 6 |
| ***GDF2*** | ENSG00000263761 | 7 |
| ***KCNA5*** | ENSG00000130037 | 8 |
| ***KCNK3*** | ENSG00000171303 | 9 |
| ***KLF2*** | ENSG00000127528 | 10 |
| ***SMAD1*** | ENSG00000170365 | 11 |
| ***SMAD4*** | ENSG00000141646 | 12 |
| ***SMAD9*** | ENSG00000120693 | 13 |
| ***TOPBP1*** | ENSG00000163781 | 14 |
| ***TBX4*** | ENSG00000121075 | 42 |

Within the last years genes belonging to the BMPR2/Alk1 -signalling pathway have been detected by whole exome next generation sequencing (NGS) to be involved in the pathogenesis of PAH, pulmonary veno-occlusive disease (PVOD) and/or HHT. In hereditary PVOD cases 100% of hereditary patients carried two mutations of the gene *EIF2AK4* (Girerd *et al.,* 2016). The ERS/ESC guidelines thus suggest a mutation analysis to be preferred to a biopsy to ascertain the diagnosis of PVOD (Galiè *et al.,* 2016). Defects in genes apart from *BMPR2* leading to PAH manifestation have been described so far in 0.5-4% of PAH patients (Machado *et al.,* 2015; Soubrier *et al.,* 2013). A mutation in the gene *KLF2* has been shown by the inventors to cause hereditary PAH (HPAH) (Eichstaedt *et al.,* 2017) and mutations in the gene *TBX4* have been identified in several cases of paediatric PAH, idiopathic PAH (IPAH) and HPAH (Levy *et al.,* 2016). Moreover, mutations in two additional genes are now considered responsible for the onset of HHT: the bone morphogenic protein 9 (*BMP9* or *GDF2*) (Wooderchak *et al.,* 2013) and the SMAD Family member 4 (*SMAD4*) (Torring *et al.,* 2014). HHT often lead to pulmonary arterial hypertension which drastically worsens patients' survival (Lyle *et al.,* 2016).

The inventors have further found that the following set of 43 genes, which comprise the 14 "core genes" (in grey), as well as the set of 15 genes, and further genes are even more preferred for the diagnosis of a genetic predisposition to PAH, PVOD, HHT and partly CTEPH:

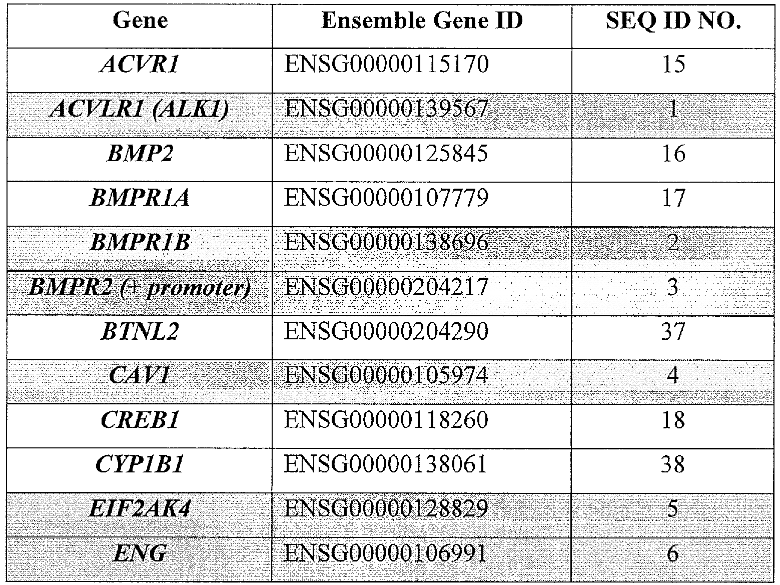

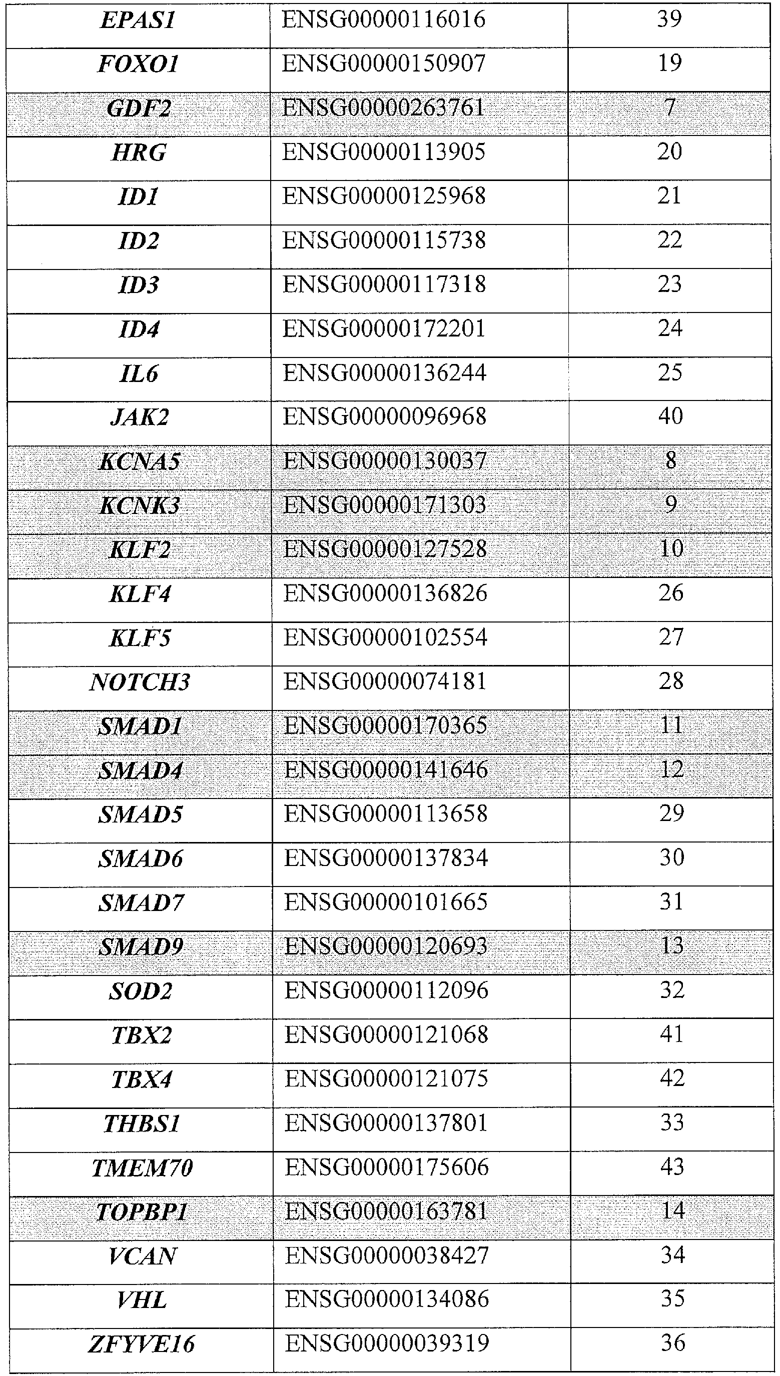

The genes *ACVR1, BMP2, BMPR1A, ID1, ID2, ID3, ID4, SMAD5, SMAD6,* and *SMAD7* (SEQ ID NOs. 15-17, 21-24, 29-31) are part of the BMPR2 pathway.

As discussed above, the genes *ACVRL1, BMRP2, ENG* (SEQ ID NOs. 1, 3, 6) have been found to be mutated in CTEPH.

As discussed above, the gene *EIF2AK4* (SEQ ID NO 5) has been shown to be mutated in PVOD.

As discussed above, the genes *ACVRL1, BMPR2, ENG, GDF2, SMAD4* (SEQ ID NOs. 1, 3, 6, 7, 12) have been shown to be mutated in HHT.

The genes *CREB1, FOXO1, HRG, IL6, KLF4, KL5, VCAN,* and *ZFYVE16* (SEQ ID NOs. 18-20, 25-27, 34, 36) are known from PAH animal models.

The gene *NOTCH3* (SEQ ID NO. 28) is involved in pediatric PAH manifestation.

The gene *SOD2* (SEQ ID NO. 32) is known from a PH animal model and is epigenetically changed in PAH patients and PH animal model.

The gene *THBS1* (SEQ ID NO. 33) is involved in hereditary PAH (HPAH) manifestation.

The gene *VHL* (SEQ ID NO. 35) is known from a PH animal model and involved in Chuvash-associated PAH (Chuvash-APAH) manifestation.

The gene *BTNL2* (SEQ ID NO. 37) is involved in sarcoidosis associated PAH.

The gene *CYP1B1* (SEQ ID NO. 38) has a decreased expression in *BMPR2* mutation carriers.

The gene *EPAS1* (SEQ ID NO. 39) is involved in HPAH manifestation and is associated with erythrocytosis.

The gene *JAK2* (SEQ ID NO. 40) is involved in myeloproliferative disease (MDS) and chronic thromboembolic pulmonary hypertension (CTEPH).

The genes *TBX2* and *TBX4* (SEQ ID Nos. 41-42) are involved in pediatric PAH manifestation associated with microcephaly thyroid and sensorineural abnormalities or small patella syndrome.

The gene *TMEM70* (SEQ ID NO. 43) is involved in persistent neonatal PAH with ATPase synthase deficiency.

In one embodiment the following set of 36 genes, which comprise the 14 "core genes" (in grey) and further genes are preferred for the diagnosis of a genetic predisposition to PAH and partly CTEPH, PVOD and/or HHT:

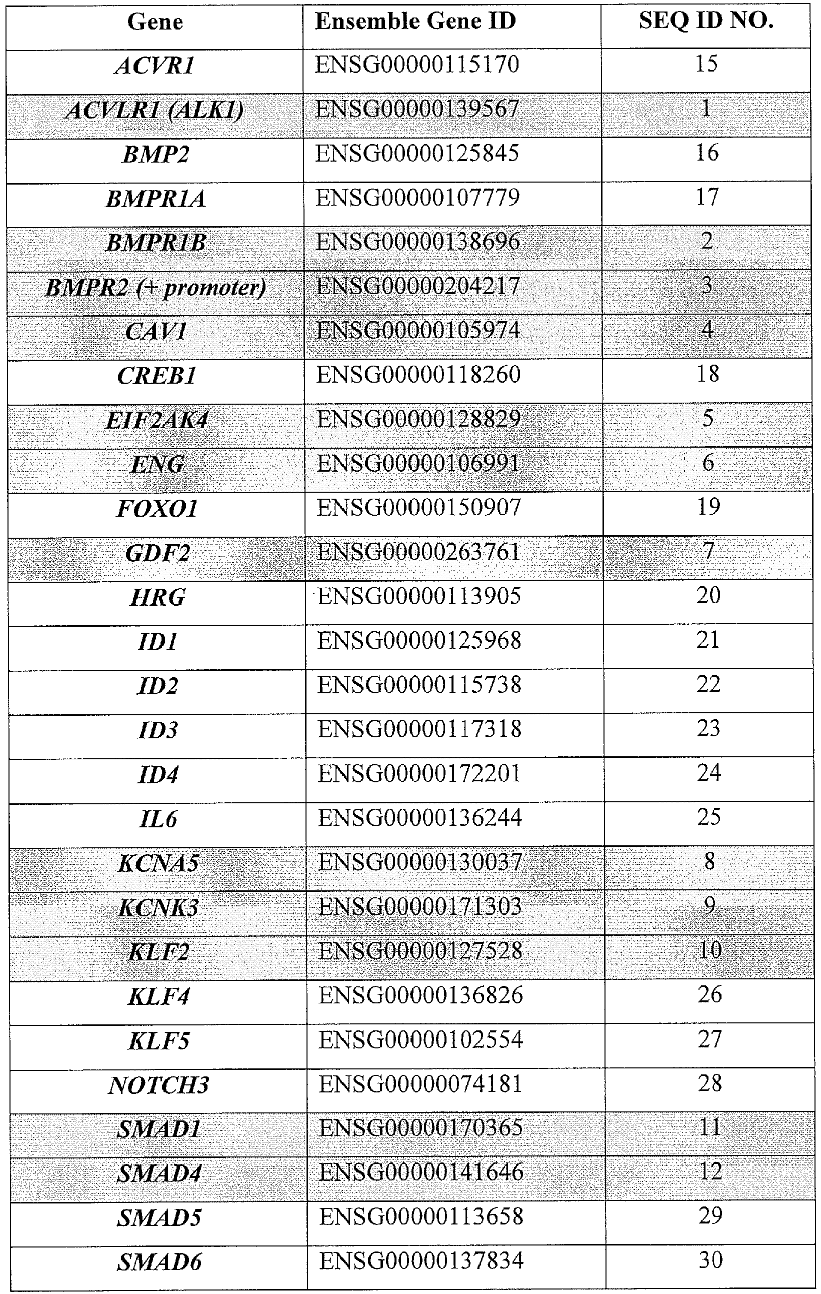

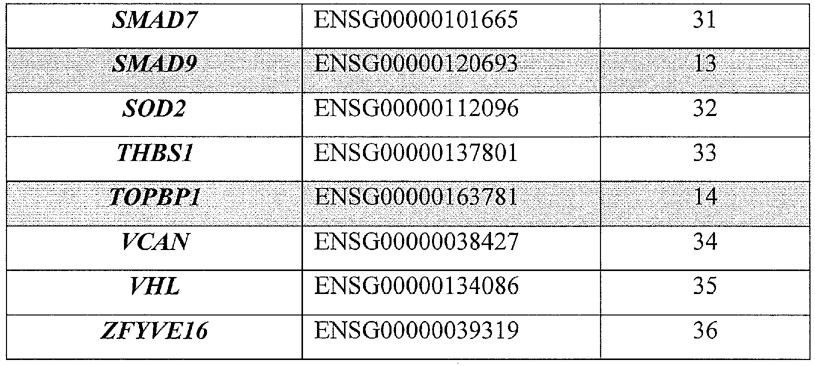

The diagnosis of genetic defects / a genetic predisposition comprises sequencing of target genomic regions of the genes of the gene panel, which can be the exons (preferably all the exons), exon-intron boundaries of the genes, and/or base pairs at the 5' and 3' untranslated region (UTR).

Said sequencing is preferably carried out or comprises next generation sequencing.

"Next generation sequencing" refers to high-throughput sequencing based on different technologies, which enable the generation of a great amount of sequence data in a short time frame opposed to the conventional, slower and more expensive direct Sanger sequencing.

Next-generation sequencing (NGS), also known as high-throughput sequencing, include e.g. the following sequencing technologies:
- Illumina (Solexa) sequencing
- Roche 454 sequencing
- Ion torrent: Proton / PGM sequencing
- SOLiD sequencing

These recent technologies allow to sequence DNA and RNA more quickly and cheaply than the previously used Sanger sequencing.

In one embodiment, the diagnosis optionally further comprises an additional sequencing, preferably Sanger sequencing, or an analysis technique, preferably multiplex ligation-dependent probe amplification (MLPA) or quantitative polymerase chain reaction (quantitative PCR).

In embodiments where large(r) deletions or insertions in the PAH/CTEPH genes are determined, an additional analysis technique (such as MLPA or quantitative PCR) is useful for confirmation.

The sample(s) is/are patient sample(s) or sample(s) from a relative of a PAH/CTEPH patient or a PAH/CTEPH/PVOD/HHT patient, respectively.

The terms "PAH/CTEPH patient" and "PAH/CTEPH/PVOD/HHT patient" are used interchangeably throughout the specification.

In one embodiment, at least two (patient) samples are tested simultaneously. A (patient) sample is preferably whole blood, peripheral blood or tissue.

In further embodiments a higher number of (patient) samples are tested, such as up to e.g. 48 samples, 96 samples or up to 384 samples. The number of samples depends also on the type of sample plate used as well as on the type of sequencer.

The gene panel or combination of genes of the present invention can preferably be used for diagnostic genetic testing in subjects, preferably in PAH/CTEPH/PVOD/HHT patients and/or its family members.

Said diagnosis includes determining whether one mutation in the respective genes is present or a second mutation in terms of one or more altered base pairs, smaller and larger deletions or insertions, wherein said mutation(s) is/are genetic confirmation of manifest or existing PAH or indicative for the increased probability for PAH/CTEPH/PVOD/HHT to occur or manifest in not yet affected family members of PAH/CTEPH/PVOD/HHT patients.

Patients with mutations have been shown to have more severe symptoms and shorter survival (Evans *et al.,* 2016), and thus these patients may be checked on their clinical symptoms in shorter intervals and their family members may be invited for genetic counselling and testing.

The gene panel or combination of genes of the present invention can preferably be used for screening of newborns, such as of babies/newborns of patients with PAH/CTEPH/PVOD/HHT or their family members, but also in a general newborn screening. The early detection of mutations may enable an early diagnosis and treatment as soon as symptoms occur.

The gene panel or combination of genes of the present invention can preferably be used for pre-implantation diagnostics, as currently offered in e.g. France (Girerd *et al.,* 2015).

In particular in families, in which the disease penetrance is high, i.e. in which many mutation carriers develop the disease, pre-implanation diagnostics may be applied within the legal framework.

### Diagnosis methods

As discussed above, the present invention provides a method for the diagnosis pulmonary arterial hypertension (PAH) and/or chronic thromboembolic pulmonary hypertension (CTEPH), in particular for the diagnosis of genetic defects in PAH and/or CTEPH.

Preferably, the diagnosis of genetic defects in pulmonary arterial hypertension comprises the diagnosis for pulmonary veno-occlusive disease (PVOD) and/or hereditary haemorrhagic telangiectasia (HHT).

Said method comprises the following steps:
(a) providing at least one sample,
   preferably at least two, preferably up to 96 samples,
(b) extracting the genomic DNA from said sample(s),
(c) determining whether at least one mutation in terms of one or more altered base pairs, smaller and larger deletions or insertions in the genes with the nucleotide sequence of SEQ ID Nos. 1 to 14 is present by using next generation sequencing, wherein the presence of said at least one such mutation is a genetic confirmation of existing PAH/CTEPH or indicative for the increased probability for PAH/CTEPH to manifest in not yet affected relatives of PAH/CTEPH patients,
   and
(d) optionally, determining whether detected mutations/gene variants have pathological consequences either predicted with *in silico* prediction programmes and/or functional studies.

The sample(s) provided in step (a) is/are patient sample(s) or sample(s) from a relative of a PAH/CTEPH patient or a PAH/CTEPH/PVOD/HHT patient, respectively.

Preferably, the (patient) sample is whole blood, peripheral blood or tissue.

As discussed above, the terms "PAH/CTEPH patient" and "PAH/CTEPH/PVOD/HHT patient" are used interchangeably throughout the specification.

In one embodiment, in step (c) the presence of at least one mutation in terms of one or more altered base pairs, smaller and larger deletions or insertions in the 14 genes with the nucleotide sequence of SEQ ID Nos. 1 to 14 and in up to 50 genes is determined, wherein said 50 genes comprise the genes with the nucleotide sequence of SEQ ID Nos. 1 to 43. Preferably, a range of at least 14 genes and up to 50 genes are used. In one embodiment 15 genes are used, in one embodiment 36 genes are used, in one embodiment 43 genes are used.

In a preferred embodiment, in step (c) the presence of at least one mutation in terms of one or more altered base pairs, smaller and larger deletions or insertions in the genes with the nucleotide sequence of SEQ ID Nos. 1 to 14 and 42.

In one embodiment, in step (c) the presence of at least one mutation in terms of one or more altered base pairs, smaller and larger deletions or insertions in the genes with the nucleotide sequence of SEQ ID Nos. 1 to 36 is determined.

In a preferred embodiment, in step (c) the presence of at least one mutation in terms of one or more altered base pairs, smaller and larger deletions or insertions in the genes with the nucleotide sequence of SEQ ID Nos. 1 to 43 is determined.

In one embodiment, the presence of at least two mutations in terms of one or more altered base pairs, smaller and larger deletions or insertions is indicative for the existence of PAH or the increased probability for PAH to become manifest.

In one embodiment, in step (b) the genomic DNA is extracted from peripheral blood using procedures known in the art.

Preferably, at least two (patient) samples are tested simultaneously. In further embodiments a higher number of (patient) samples are tested, such as up to e.g. 48 samples, 96 samples or up to 384 samples. The number of samples depends also on the type of sample plate used as well as on the type of sequencer.

The genomic DNA can be tagged, such as with a barcode. The DNA of each patient / each subject can be tagged differently allowing multiple samples to be tested in parallel / simultaneously.

The genomic DNA can be digested e.g. with restriction enzymes and the desired genes can be captured by 50 to 500 bp biotinylated probes annealing to the respective genomic sequence. The pull-down of desired fragments can be achieved via biotin-streptavidin beads.

The fragments can be amplified via PCR and loaded onto a sequencer for sequencing.

In one embodiment, the method comprises the further step of
(d) carrying out an additional sequencing,
preferably Sanger sequencing,
or an analysis technique,
preferably multiplex ligation-dependent probe amplification or quantitative polymerase chain reaction.

In embodiments where large(r) deletions or insertions in the PAH/CTEPH/PVOD/HHT genes are determined, an additional analysis technique (such as MLPA or quantitative PCR) is useful for confirmation.

In one embodiment, the method is used for
- diagnostic genetic testing in subjects, preferably in PAH/CTEPH/PVOD/HHT patients and/or its family members;
- screening of newborns; and/or
- pre-implantation diagnostics;

### Diagnostic PAH kit

As discussed above, the present invention provides a kit for the diagnosis of pulmonary arterial hypertension (PAH) and/or chronic thromboembolic pulmonary hypertension (CTEPH), in particular for the diagnosis for a genetic predisposition to PAH and/or CTEPH.

Preferably, the diagnosis of genetic defects in pulmonary arterial hypertension comprises the diagnosis for pulmonary veno-occlusive disease (PVOD) and/or hereditary haemorrhagic telangiectasia (HHT).

Said kit comprises
(i) means for carrying out next generation sequencing and detecting at least one mutation in terms of one or more altered base pairs, smaller and larger deletions or insertions in the genes with the nucleotide sequence of SEQ ID Nos. 1 to 14.

In one embodiment, the means (i) are means for carrying out next generation sequencing and detecting at least one such mutation in the genes with the nucleotide sequence of SEQ ID Nos. 1 to 14 and 42.

In one embodiment, the means (i) are means for carrying out next generation sequencing and detecting at least one such mutation in the genes with the nucleotide sequence of SEQ ID Nos. 1 to 36, preferably in the genes with the nucleotide sequence of SEQ ID Nos. 1 to 43.

Said means comprise probes suitable for sequencing target genomic regions of the respective genes and for determining whether at least one such mutation in said genes is present.

Said target genomic regions can be the exons (preferably all the exons), exon-intron boundaries of the genes, and/or base pairs at the 5' and 3' untranslated region (UTR).

Said probes can have a length of 50 to 500 nucleotides. Said probes can be labelled, such as via biotin, and/or fluorophores.

Said means can, for example, be designed and generated using HaloPlex technology or SureSelectXT technology (Agilent Technologies, Santa Clara, USA) or using the *in silico* tool SureDesign (Agilent Technologies, Santa Clara, USA).

Said kit can optionally further comprise
(ii) means for processing the genomic DNA.

Said processing means can be for tagging, digesting, capturing etc.

The genomic DNA can be tagged, such as with a barcode. The DNA of each patient can be tagged differently allowing multiple samples to be tested in parallel / simultaneously.

The genomic DNA can be digested with e.g. restriction enzymes and the desired genes can be captured by 50 to 500 bp biotinylated probed annealing to the respective genomic sequence. The pull-down of desired fragments can be achieved e.g. via biotin-streptavidin beads.

The fragments can be amplified via PCR and loaded onto a sequencer for sequencing.

Said kit optionally further comprises
(iii) means for carrying out an additional sequencing,
preferably Sanger sequencing,
or for carrying out an analysis technique,
preferably multiplex ligation-dependent probe amplification or quantitative polymerase chain reaction.

Said means (iii), for example, comprise dNTPs, polymerases, buffer, magnesium chloride, specific primers and probes.

In embodiments where large(r) deletions or insertions in the PAH/CTEPH/PVOD/HHT genes are determined, an additional analysis technique (such as MLPA or quantitative PCR) is useful for confirmation.

### Preferred embodiments

### - Abstract

In this study we developed a new specific gene panel for pulmonary arterial hypertension (PAH), chronic thromboembolic pulmonary hypertension (CTEPH), pulmonary veno-occlusive disease (PVOD) and hereditary haemorrhagic telangiectasia (HHT) including major disease genes and further candidates. The new PAH-specific gene panel developed in this study allowed for the first time the assessment of all known PAH genes and further candidates at once and markedly reduced overall sequencing time and costs. Sensitivity and specificity reached 100% when Sanger technique was additionally applied. Thus, this technique will potentially change the routine diagnostic genetic testing in PAH patients.

### - Discussion

We developed a PAH/CTEPH/PVOD/HHT-specific gene panel based on NGS and tested sensitivity and specificity including 43 PAH/CTEPH/PVOD/HHT genes and candidates in up to 96 patients and family members. A pilot study, including 29 genes, revealed *BMPR2, ALK1, ENG* and *EIF2AK4* mutations in 59% of patients and documented a 100% sensitivity and 100% specificity of the panel, when Sanger technique was additionally applied in a "two step" procedure. The study showed that mutations in "new" genes such as *EIF2AK4* could be frequently detected in PAH patients which would have been overlooked by the commonly used standard procedure addressing the 3 major PAH/CTEPH/PVOD/HHT genes (*BMPR2, ALK1, ENG*) only. Therefore, this technique will completely change the usual molecular diagnostic approach. NGS enables the screening of many genomic regions at once, Sanger sequencing of the pathologic variants as second step enables a 100% specificity.

Very recently another study was published by Piao and colleagues (2016) investigating mutations in 22 PAH associated genes in a cohort of IPAH/HPAH patients. They could only identify mutations in the genes *BMPR2* and *ALK1* in 25% of the patients. No mutations in other candidate genes were uncovered. While 11 genes overlapped with our panel we also present data for 34 distinct candidate genes. *EIF2AK4* was not sequenced by Piao *et al.* and thus potential mutations might have been overlooked. This exemplifies how crucial the selection of candidate genes is to detect PAH causing mutations.

### Technical advantages of the new PAH/CTEPH-specific gene panel as diagnostic tool

The PAH/CTEPH-specific gene panel is much more time and cost efficient than the conventional Sanger sequencing. The time per patient can be drastically reduced even further with different hardware analysing up to 96 patients at once. The number of genes could be increased up to 50, albeit lowering the average coverage of analysed genes.

The newly developed panel generates results for all genes in the same process. In contrast, Sanger sequencing is performed sequentially, one gene at a time. Hence, Sanger sequencing would be terminated as soon as a single mutation was identified excluding the possibility of detecting additional mutations as second hits (Rodriguez Viales *et al.,* 2015). Therefore, a similar NGS panel approach is already in use for common diseases such as familiar breast and ovarian cancer (Schroeder *et al.,* 2015).

Alternatively to Sanger technique whole exome NGS has been previously applied in PAH patients and detected 4 new PAH genes: the membrane protein caveolin-1 (Austin *et al.,* 2012), the potassium channel *KCNK3,* the translation factor *EIF2AK4* and the topoisomerase binding protein *TOPBP1* (de Jesus Perez *et al.,* 2014). However, whole exome NGS requires such an enormous amount of work (approximately several months to identify putative PAH genes in one patient) in terms of bioinformatics analyses, data processing and storage that this technique is currently no option for the routine diagnostic setting. It is only feasible for scientific use.

### Further methodological considerations

Additional 5 candidate genes identified with conventional methods included *BMPR1B* (Chida *et al.,* 2012), *KCNA5* (Remillard *et al.,* 2007), *SMAD1* (Nasim *et al.,* 2008), *SMAD4* (Nasim *et al.,* 2008) and *SMAD9* (Shintani *et al.,* 2009). These genes represent the most promising candidates to be mutated in IPAH/HPAH patients with no mutations in *BMPR2, ENG* or *ALK1* (Kwapiszweska *et al.,* 2014). Hence, a targeted screening of all of these genes increases the probability of a disease causing mutation to be detected. Moreover, the continuous inclusion of putative candidate genes in the same panel will advance our knowledge of the PAH aetiology and highlight new genes involved in disease manifestation. The new PAH/CTEPH/PVOD/HHT-specific gene panel presented in this application will allow to better identify additional mutations, acting as modifiers for disease manifestation.

Genetic assessment might receive a more prominent role within the actual and future diagnostic algorithm as indicated in the new ESC/ERS-guidelines and may even be useful for risk stratification. At the same time, the use of the PAH/CTEPH/PVOD/HHT-specific gene panel diagnostic requires a more comprehensive genetic counselling before and after the molecular analysis, since more genes are analysed and variants of unknown significance will also have to be explained. Once mutations have been identified in PAH/CTEPH/PVOD/HHT patients the genetic assessment and counselling of family members is furthermore complicated by the yet unknown penetrance of mutations in less common PAH genes (Girerd *et al.,* 2016).

### Mutations and variants of uncertain significance

Both newly identified heterozygous mutations of the pilot study were located within *EIF2AK4.* Mutations in *EIF2AK4* have been previously described in families with PVOD (Eyries *et al.,* 2014) and pulmonary capillary haemangiomatosis (Best *et al.,* 2014). Whereas we included one patient with PVOD this patient showed no mutation or variant in *EIF2AK4* but a *BMPR2* mutation. Recently, mutations in *EIF2AK4* were also identified in 5 families of an Iberian population suffering from an aggressive form of HPAH (Tenorio *et al.,* 2014) and two additional families (Gomez *et al.,* 2015). Thus, this is the third study reporting mutations of *EIF2AK4* in I/HPAH-patients.

Twelve variants were classified as variants of unknown significance in a pilot study analysing a subset of 29 genes in 37 patients and 5 family members of other PAH patients since not all *in silico* prediction programmes characterised them as "pathogenic" and/or they were present in a public database. Nevertheless, variants, which were classified as "pathogenic" by at least one prediction programme and are absent in non-PAH patients, represent interesting follow-up candidates for functional studies such as the variants in exon 23 of *EIF2AK4,* in exon 3 of *SMAD7* or in *SMAD6* exon 4. Due to the low frequency of PAH in the population (15:1,000,000) already two individuals with the same variant in the public ExAC database rise the population frequency of the variant above 0.0015%, the conservative cut-off expected for mutations causing PAH (Machado *et al.,* 2015). Considering this level, a variant at a frequency of 0.0016%, which occurs in two individuals in the database, such as the variant in exon 3 of the *VCAN* gene might still represent an interesting follow-up candidate.

### Conclusions

The new approach of a panel diagnostic including a vast array of PAH/CTEPH/PVOD/HHT associated genes has shown to have a high sensitivity and specificity together with confirmatory Sanger sequencing. In already a small subset of patients two mutations in *EIF2AK4* were identified, which would have not been detected with the previous routine procedure. Therefore, for patients and for practitioners this approach offers a comprehensive, cost and time efficient way to investigate genetic influences of PAH/CTEPH/PVOD/HHT and can be continuously adapted to include newly detected causative PAH/CTEPH/PVOD/HHT genes.

### Further preferred embodiments

1) The inventors have found evidence that in some patients at least 2 gene defects are necessary for disease manifestation (second hit hypothesis) (Eichstaedt *et al.,* 2016; Rodriguez Viales *et al.,* 2015).

### - Abstract

Mutations in the *EIF2AK4* gene have recently been identified in recessively inherited VOD. In this study we assessed if *EIF2AK4* mutations occur also in a family with autosomal dominantly inherited pulmonary arterial hypertension (HPAH) and incomplete penetrance of *BMPR2* mutations.

Clinical examinations in a family with 10 members included physical examination, electrocardiogram, (stress)-echocardiography and lung function. Manifest PAH was confirmed by right heart catheterisation in three affected subjects. Genetic analysis was performed using a new PAH-specific gene panel analysis with next generation sequencing of all known PAH and further candidate genes. Identified variants were confirmed by Sanger sequencing.

All living family members with manifest HPAH carried two pathogenic heterozygous mutations: a frame shift mutation in the *BMPR2* gene and a novel splice site mutation in the *EIF2AK4* gene. Two family members who carried the *BMPR2* mutation only did not develop manifest HPAH,

This is the first study suggesting that *EIF2AK4* can also contribute to autosomal dominantly inherited HPAH. Up to now it has only been identified in a recessive form of HPAH. Only those family members with a co-occurrence of two mutations developed manifest HPAH. Thus, the *EIF2AK4* and *BMRPR2* mutations support the "second hit" hypothesis explaining the variable penetrance of HPAH in this family. Hence, the assessment of all known PAH genes in families with a known mutation might assist in predictions about the clinical manifestation in so far non-affected mutation carriers.

### - Discussion

This is the first report of an autosomal dominantly inherited *EIF2AK4* mutation as second hit in a family with HPAH and known *BMPR2* mutation. Only those family members with a co-occurrence of a mutation in *BMPR2* and *EIF2AK4* were clinically affected and developed manifest HPAH, whereas carriers of the *BMPR2* mutation only had no symptoms of PAH. Thus, the results of this study offer an explanation for the reduced penetrance of the disease in this family and show that *EIF2AK4* may play a role also in families with autosomal dominantly inherited PAH.

### EIF2AK4 mutation in autosomal dominant HPAH

*EIF2AK4* was first described in the autosomal recessively inherited pulmonary veno-occlusive disease (PVOD) (Eyries *et al.,* 2014) and pulmonary capillary haemangiomatosis (Best *et al.,* 2014). Recently a single recessively inherited *EIF2AK4* mutation (c.3344C>T, p.(P115L)) was repeatedly identified in six consanguineous HPAH families with autosomal recessive mode of inheritance (Gomez *et al.,* 2014; Tenorio *et al.,* 20105). Only homozygous mutation carriers developed the disease plus a single heterozygous carrier of a distinct *EIF2AK4* mutation, in whom the authors suspected a second non-identified mutation in the same gene (Gomez *et al.,* 2015). Therefore, up to now *EIF2AK4* mutations have been believed to be a very rare in HPAH. Apart from the mentioned family we were able to identify a nonsense mutation in exon 8 in the *EIF2AK4* gene in another PAH patient with sporadic IPAH who had no other mutation in known candidate genes (data not shown). Thus, this gene might be more often affected than initially thought and contributes to the disease in an autosomal dominant manner. In contrast, *BMPR2* mutations occur in up to 85% of familial cases and are autosomal dominantly inherited (Machado *et al.,* 2015; Pfarr *et al.,* 2011). However, many *BMPR2* gene carriers have no clinical symptoms and do not develop manifest PH even during a more than 10 year follow-up period (Hinderhofer *et al.,* 2014). Hence, the family described here provides an explanation for the decreased penetrance and suggests an autosomal dominantly contribution of *EIF2AK4* to disease manifestation.

### EIF2AK4 mutation as "second hit" may explain variable penetrance

Up to date only four families with second hits have been described (Rodriguez Viales *et al.,* 2015; Wang *et al.,* 2014). This might be due to the fact that usually only 3 genes (*BMPR2, ACVRL1, ENG*) are analyzed routinely in PAH patients in a sequential processes, i.e. if one mutation is discovered the other genes are not assessed. Thus, second hit mutations might be overlooked in general in the current routine diagnostic setting and particularly in genes such as *EIF2AK4,* which are usually not included in the diagnostic analysis.

While second hits are still rarely described in PAH this model is often found in other diseases such as the nephrotic syndrome or the long QT-syndrome. At the same time the decreased penetrance in PAH is an acknowledged pattern. Thus, second hits or modifier genes might be more common in PAH than known to date. We therefore contrast two genetic models: Firstly, we propose the "second hit model" to explain the low disease penetrance in PAH. In this model a single mutation in each gene on its own has a very low penetrance. Two mutations however, lead to a synergistic effect resulting in a high disease penetrance. The second model is the "single gene model", representing the classical view for PAH suggesting *BMPR2* mutations alone are responsible for disease manifestation. Under this assumption, the *EIF2AK4* mutation would randomly occur in this family and not impact PAH manifestation. In the latter model the penetrance for the *BMPR2* variant must be moderate, since 4 (obligate) carriers of the mutation did not develop PAH up to ages 31, 37, 49 and 59.

Considering both models, it is significantly unlikely that both mutations in two known PAH genes occurred by chance in this family. Moreover, the *EIF2AK4* mutation clearly co-segregates with the disease in *BMPR2* positive family members suggesting a second hit model.

We have not observed an *EIF2AK4* mutation alone within this family, albeit in a different IPAH patient (data not shown) indicating at least a low penetrance to be present. Disease severity might moreover be influenced by the location of the respective mutations within the protein and thus their variable impact on protein function (Machado *et al.,* 2015). Therefore, we hypothesize according to the second hit model the penetrance to be intermediate if only *BMPR2* was positive, very low if only *EIF2AK4* was positive, and very high if *EIF2AK4* and *BMPR2* each harbor a mutation.

### Loss of protein function by EIF2AK4 mutation

The gene *EIF2AK4* encodes a kinase termed general control nonderepressable 2 (GCN2) which phosphorylates the eukaryotic translation initiation factor 2α leading to a global down regulation of protein synthesis in response to amino acid starvation, hypoxia and viral infection but the up-regulation of specific stress response proteins (Montani *et al.,* 2016). Gene expression is increased in smooth muscle cells in the vessel wall and interstitial tissue (Eyries *et al.,* 2014). While the gene function has been studied a clear link to PVOD or PAH still remains to be detected. However, an interaction between *EIF2AK4* and the BMPR2 pathway genes *SMAD1, SMAD4, ACVRL1* and *ENG* has been observed (Barrios-Rodiles *et al.,* 2005; Eyries *et al.,* 2014). Thus, an impaired functioning of both, BMPR2 and GCN2 (*EIF2AK4*), might potentiate its effect on the transcription of target genes of the BMPR2 pathway.

The *EIF2AK4* mutation of this family leads to the loss of a splice site and subsequently the loss of exon 38, presumably a frame shift and premature stop codon. In the last exons of the functional gene (31-39) lies the ribosomal binding domain and the dimerisation domain between amino acids 1396-1643 (Padayana *et al.,* 2005). The last 51 amino acids of this domain were missing or partly exchanged by wrong amino acids in the affected members of this HPAH family. The domain is essential to recruit ribosomes for protein synthesis (Narasimhan *et al.,* 2004), thus a partial deletion will at least moderately affect protein-ribosome binding if not fully impair it. Moreover, in the same region the dimerisation domain is located. This facilitates the formation of a homodimer (2 EIF2AK4 proteins bind to each other) and thus a functional protein (Narasimhan *et al.,* 2004). The formation of homodimers has been shown to be conserved in mice and yeast (He *et al.,* 2014). Single amino acid substitutions in the C-terminal domain in yeast already led to an inability of the protein to dimerise and to be functionally active (Narasimhan *et al.,* 2004). The gene sequence of the C-terminal domain is highly conserved from mice to mammals suggesting corresponding functional impairments in humans (Berlanga *et al.,* 1999). A total deletion of this region in our HPAH family thus likely leads to a loss of function in the mutated gene.

### Conclusions

We were able to show *EIF2AK4* contributes to disease manifestation in this HPAH family in an autosomal dominant manner. We report a new mutation within *EIF2AK4* leading to HPAH as a second hit together with a mutation in *BMPR2* providing an explanation for the observed penetrance in this family. Only those family members with a co-occurrence of two mutations developed manifest HPAH. The occurrence of several mutations in PAH associated genes might be more frequent than initially thought. Thus, a simultaneous assessment of all PAH associated genes in more patients might shed light on the long standing question surrounding the reduced penetrance.

2) A mutation in the gene *KLF2* has been shown by the inventors to cause hereditary PAH (HPAH) (Eichstaedt *et al.,* 2017).

### -Abstract

Heritable pulmonary arterial hypertension (HPAH) is an autosomal dominantly inherited disease caused by mutations in the *BMPR2* gene and/or genes of its signalling pathway in approximately 85% of patients. We clinically and genetically analysed an HPAH family without mutations in previously described PAH genes. Clinical assessment included electrocardiogram, lung function, blood gas analysis, chest X-ray, laboratory testing, echocardiography and right heart catheterization in case of suspected disease. Genetic diagnostics were performed using a PAH-specific gene panel including all known 12 PAH genes and 20 further candidate genes by next-generation sequencing (NGS). HPAH was invasively confirmed in two sisters and their father who died aged 32 years. No signs of HPAH were detected in five first-degree family members. Both sisters were lung transplanted and remained stable during a follow-up of >20 years. We detected a novel missense mutation in the *Krüppel-like factor 2* (*KLF2*) likely leading to a disruption of gene function. The same *KLF2* mutation has been described as a recurrent somatic mutation in B-cell lymphoma. Neither the healthy family members carried the mutation nor >120000 controls. These findings point to *KLF2* as a new PAH gene.

### - Discussion

In this study we detected a novel germline mutation in the *KLF2* gene likely causing autosomal inherited HPAH in a family with three affected patients, a follow-up of >20 and no mutations in any of the previously described 12 PAH genes. The identification of *KLF2* as potential PAH gene will generate new insights into the pathogenesis of the disease since it regulates pathways which have been shown to be involved in PAH development such as cell proliferation, inflammation and vasodilation.

### Is the KLF2 gene mutation disease causing in this family?

*KLF2* is a promising PAH gene, as Krüppel-like factors play an important role in many cellular functions which are also involved in the disease aetiology of HPAH. The pathogenicity of the detected *KLF2* mutation was supported by several levels of evidence. Firstly, the functional spectrum of the KLF2 transcription factor, in particular its implication in endothelial activation and proliferation (Bhattacharya *et al.,* 2005) and regulation of vasotonus points to an involvement in the development of PAH. Since KLF2 is able to inhibit a driver of endothelial cell proliferation, the VEGF receptor 2 (Bhattacharya *et al.,* 2005), mutations which impair KLF2 functioning, may also lead to an increased cell proliferation. Similarly, functional KLF2 downregulates vasoconstrictors such as endothelin-1 and upregulates vasodilators such as eNOS (Dekker *et al.,* 2005). Hence, a disturbance in this regulation is likely increasing vascular tonus, which is also observed in PAH.

Secondly, the mutation is significantly associated with PAH and co-segregated with the disease in our family. It has not been described before in PAH and was absent in unaffected family members as well as more than 120,000 controls.

Thirdly, the same mutation has been identified in splenic marginal zone lymphoma (Clipson *et al.,* 2015) and functional studies clearly indicated a displacement from the nucleus and reduced transcriptional activity (Piva *et al.,* 2015). The lymphoma-associated mutations have been identified as somatic mutations, whereas the presumption in this study is that the *KLF2* mutation is a germline event and thus present during development. This is a critical distinction, as homozygous germline deletion of *KLF2* in mice has been published to be embryonic lethal or at least very deleterious to normal embryonic development (Yeo *et al.,* 2014). The mechanism of embryonic lethality relates in part to loss of vascular integrity, lending some additional biological plausibility to *KLF2* as a PAH-related gene. However, the published data suggest a specific activity of the mutant that is strongly reduced compared to the wild-type (Piva *et al.,* 2015). This would fit with an incompletely penetrant, embryonically viable mutation that nonetheless manifests in disease. Thus, considering all evidence it is highly likely that the *KLF2* mutation in this family led to PAH manifestation.

### KLF2 gene is linked to the BMPR2-signalling

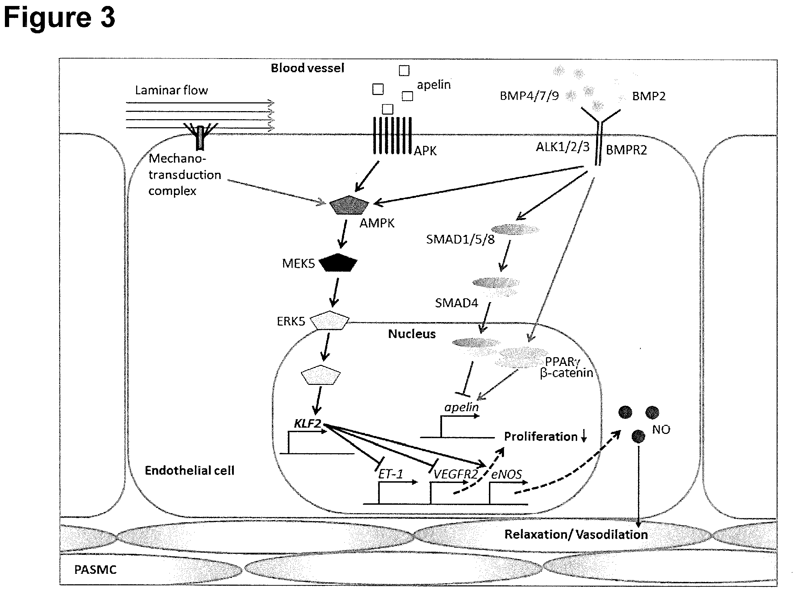

A closer review of KLF2 functionality and cellular localisation revealed a tight connection to the BMPR2 signalling pathway (Figure 3). We summarised interactions of KLF2 and important pathway members such as BMPs and apelin regulating vasodilation and cell proliferation known from the literature illustrating its role in this signalling network. KLF2 is not only upregulated by laminar blood flow in the pulmonary arteries but also by the BMPR2 ligand BMP4 and apelin, a small molecule acting as a vasodilator (Kim 2014). The functional BMPR2-apelin-KLF2 signalling axis leads to vasodilation by activating eNOS (SenBanerjee *et al.,* 2004). Reduced apelin serum levels were identified in IPAH patients in comparison to controls (Chandra *et al.,* 2011). Thus, an imbalance of the BMPR2-apelin-KLF2 axis might contribute to dysfunctional endothelial and smooth muscle cells which are observed in PAH. This offers a possible explanation for the development of HPAH in the described family.

### Conclusion

The findings of this study highlight for the first time *KLF2* as a potential PAH gene. KLF2 functions are closely tied to the disease aetiology of PAH as it regulates vasotonus, cell proliferation and endothelial activation and the gene is linked to the BMPR2 pathway.

The following examples and drawings illustrate the present invention without, however, limiting the same thereto.

### BRIEF DESCRIPTION OF THE DRAWINGS

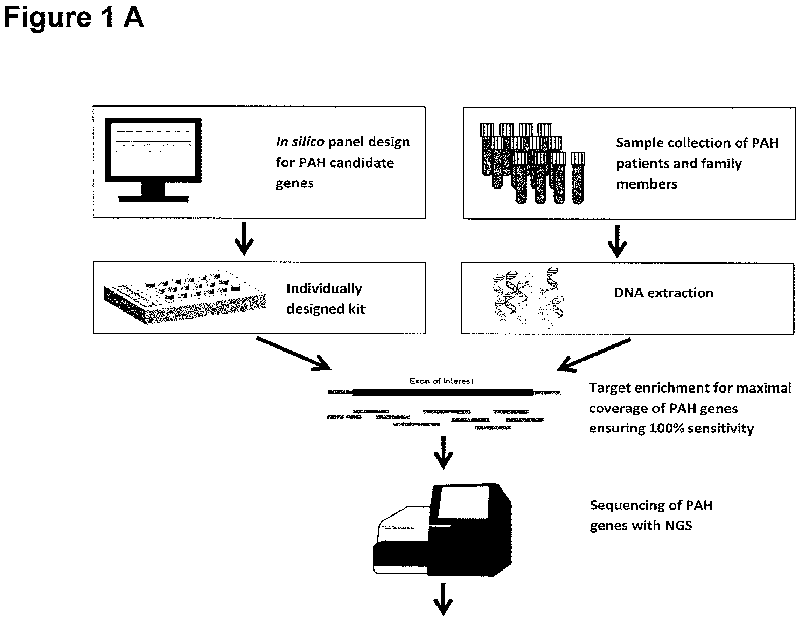

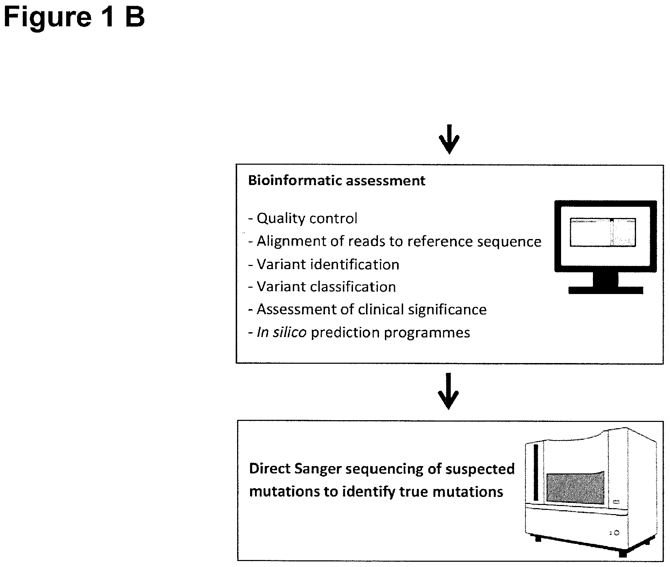

**Figure 1****.** *Two step sequencing procedure of the PAH specific gene panel diagnostic.*
   (A) In the first step the panel is designed *in silico.* The customised panel is manufactured and the desired PAH/CTEPH/PVOD/HHT genes are extracted from DNA of PAH/CTEPH patients and family members. PAH/CTEPH/PVOD/HHT genes from each patient are enriched leading to a high coverage of the regions of interest allowing 100% sensitivity to detect all true positive variants. Samples are pooled and loaded together onto a next generation sequencer.
   (B) Resulting data is passed through quality control, the reads are aligned to a reference sequence and any variant from polymorphisms to mutations is identified. These variants are assessed regarding their clinical significance with comparative data bases and *in silico* prediction programs. To eliminate false positives direct Sanger sequencing is applied in a second sequencing step reaching 100% specificity.

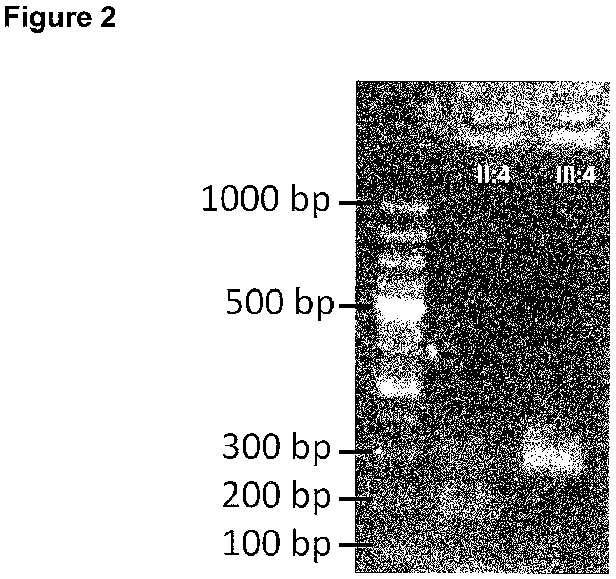

**Figure 2****.** *Effect of the EIF2AK4 mutation c.4892G>T on cDNA level.*
   Next to the ladder the affected individual II:4 shows a heterozygous PCR product for the cDNA of *EIF2AK4* exons 36-39. The upper band shows the wildtype sequence (266 bp) while the lower band shows a product without exon 38 (147 bp). The healthy family member III:4 is homozygous for the wild type PCR product indicating no loss of exon 38.
**Figure 3****.** *KLF2 as part of the BMPR2 signalling pathway.*
   KLF2 is upregulated by laminar blood flow, apelin and the bone morphogenic protein 4 (BMP4), which binds to the dimerised BMP receptor 2 (BMPR2) and the activin receptor like kinase (ALK or *ACTIRL1*)*.* Signals are transduced via the adenosine monophosphate kinase (AMPK), the mitogen activated protein kinase kinase 5 (MEK5 or *MAP2K5*) and subsequently the extracellular signal regulated kinase 5 (ERK5 or *MAPK7*)*. Apelin* transcription is down regulated by BMP4, 7 and 9 orchestrated via the sma- and mad-related (SMAD) pathway. In contrast, BMP2 is able to induce *apelin* expression via the nuclear complex beta-catenin and peroxisome proliferator-activated receptor gamma (PPARγ). Thus, the BMPR2 pathway can directly activate *KLF2* and regulate apelin (*APLN*)*,* an upstream regulator of *KLF2.* The KLF2 transcription factor inhibits endothelin-1 (ET-1 or *EDN1*) and vascular endothelial growth factor receptor 2 (VEGFR2 or KDR) expression while increasing endothelial nitric oxide synthase (eNOS or *NOS3*) transcription. These effects lead to a reduction in endothelial cell proliferation, the generation of NO and subsequent vasodilation of pulmonary artery smooth muscle cells (PASMCs). With a dysfunction of the *KLF2* gene caused by a germline mutation as detected in this study, several PAH pathways will be activated. (Eichstaedt *et al.,* 2017).

### EXAMPLES

### EXAMPLE 1

### 1. Materials and Methods

### 1.1 Subjects and clinical characterisation

In all PAH patients of the pilot study diagnosis was performed according to current ERS/ESC-guidelines. All patients underwent a detailed clinical examination including right heart catheterisation as described previously (Grünig *et al.,* 2009). Testing for vasoreactivity and left heart catheterisation were performed when clinically indicated. Clinical assessment of family members included taking medical history, lung function, six minute walking distance, physical examination, 12-lead electrocardiogram, echocardiography at rest and during supine bicycle exercise (Grünig *et al.,* 2009; Kabitz *et al.,* 2008). Acute or chronic pulmonary and cardiac diseases were ruled out in all family members. Blood samples were collected from all patients and relatives of other PAH patients. The protocol of this study confirms to the ethical guidelines of the Declaration of Helsinki. It was approved by the Ethic Committee of Heidelberg University and participants or their parents gave written informed consent.

### 1.2 Development of the PAH/CTEPH/PVOD/HHT-specific gene panel

We developed a technique which in general would allow to asses up to 50 genes in up to 96 patients/family members simultaneously. In brief, after DNA is extracted, genomic regions of interest were enriched with a self-designed customised PAH panel, sequenced with NGS and possible pathogenic variants confirmed with targeted Sanger sequencing (Figure 1).

### 1.3 Implementation of the PAH/CTEPH/PVOD/HHT-specific gene panel

The new gene panel was manually designed with the *in silico* tool SureDesign (Agilent Technologies, Santa Clara, USA). While the gene panel may assess up to up to 50 genes in 96 patients we concentrated on 43 genes and 37 patients and 5 relatives of other PAH patients to evaluate the sensitivity and specificity of this approach in the presented pilot study.

For this pilot study, a list of 29 genes including 12 PAH causal genes, was created after extensive literature research (Table 1 lists these 29 genes). The respective genomic regions, i.e. all exons plus 25 base pairs at 5' and 3' untranslated region and adjacent exon-intron boundaries, were extracted *in silico* from the human genome (GhR37/hg19). Subsequently, probes were designed which covered each single DNA base within the desired genomic regions by on average of 7 different probes. Based on the *in silico* design a customised gene panel was manufactured by Agilent Technologies (HaloPlex).

DNA from subjects was extracted from peripheral blood using standardised procedures (Qiagen, Hilden, Germany) and tagged with a bar code allowing multiple samples to be included in a single run. The library preparation *in vitro* included DNA digestion by 16 restriction enzymes and the capture of the desired target genes by biotinylated probes. After pull down of desired fragments with biotin-streptavidin beads fragments were amplified by PCR and loaded onto the MiSeq (Illumina, San Diego, USA) for subsequent sequencing. The kit may either be used entirely in one run on a NGS machine or split up in several runs. The latter option was used for the MiSeq and we performed four runs with 9-12 patients each.

### 1.4 Data analysis and setting adjustments

Data was imported into SeqPilot 4.1.2 (JSI medical systems GmbH, Kippenheim, Germany). Transcripts were loaded using the GhR37/hgl9 automated feature. Regions of interest were defined as coding exons including 20 base pairs of the 5' and 3' untranslated region. Default parameters were altered to highlighted changes in the sequence when ≥20 reads covered the specific base pair to account for non-random distributed reads over target regions in the HaloPlex design (Schroeder *et al.,* 2015).

While heterozygosity in Sanger sequencing is characterised by an equal frequency of the two variants, NGS is based on probes which amplify regions of interest. Differential amplification of specific probes might lead to a skewed ratio of the two variants. The initial heterozygosity cut-off of 35% to 65% for the two respective alleles was lowered to ≥10% and potential pathogenic variants were re-assessed with Sanger sequencing.

### 1.5 Variant classification

Each variant was classified as either clearly benign (polymorphism), likely benign, uncertain significance, likely pathogenic or clearly pathogenic as recommended by the Human Genome Variation Society (HGVS, http://www.hgvs.org/) (Richards *et al.,* 2015). Each variant was evaluated in terms of minor allele frequency (MAF) in ENSEMBL and absolute frequency in the Exome Aggregation Consortium (ExAC) including more than 60,000 exomes from unrelated individuals (Lek *et al.,* 2016). The consideration of this large database can prevent common variants from being classified as disease causing (Granzow *et al.,* 2015). Polymorphisms were defined as a MAF ≥5% (Richards *et al.,* 2015). All variants with a MAF <5% were sought in the Human Gene Mutation Database (HGMD Professional 2015.4). Additionally, for variants of *BMPR2, ENG, ALK1* and *SMAD4* present in the ARUP data base (http://www.arup.utah.edu/database/) a literature search was conducted. Non-synonymous missense variants with MAF <5% were characterised using four *in silico* prediction programmes: MutationTaster, SIFT, Align GVGD and PolyPhen2 implemented in Alamut 2.7.1 (interactive biosoftware, Rouen, France). Variants were considered as likely pathogenic in case of non-sense, frameshift and splice site mutations not occurring in the last exon of a gene (Wallis *et al.,* 2013) and are termed mutations hereafter. Variants which could not be clearly assigned with the majority of the *in silico* prediction tools were classified as having an "uncertain significance".

### 1.6 Sanger sequencing, multiplex ligand-dependent probe amplification and statistical evaluation

All variants of uncertain significance, likely pathogenic variants or mutations were reanalysed with Sanger sequencing after designing specific primers and evaluating the optimal polymerase chain reaction (PCR) conditions.

| ***Gene*** | ***Primer name*** | ***Primer sequence 5'-3'*** | ***SEQ ID NO.*** |
|---|---|---|---|
| *ACVRL1* | ACVRL1-X3 M13Fn1 | TTCAAGGTGTTTGTCTGAGGG | 44 |
| *ACVRL1* | ACVRL1-X3 P172Rn1 | CCAGAGCATGAGAGGAAAGG | 45 |
| *ACVRL1* | ACVRL1-X5 M13f | GGATAGAGAAGGGGGCTGTG | 46 |
| *ACVRL1* | ACVRL1-X5 P172rn | TAGCCAAAAACTCCCTCACC | 47 |
| *ACVRL1* | ACVRL1-X6 M13fn | CCCAGTGTGTAACCCTCACC | 48 |
| *ACVRL1* | ACVRL1-X6 P172rn | GGAGGTCTGCAAACTTGAGC | 49 |
| *ACVRL1* | ACVRL1-X7 M13fn | GCTGAGTCACCCAACCTTTC | 50 |
| *ACVRL1* | ACVRL1-X7 P172rn | AGACAACTCGTGGGGTCTTG | 51 |
| *ACVPL1* | ACVRL1-X8 M13fn | GATCCCAGGTTTGGGAGAG | 52 |
| *ACVRL1* | ACVRL1-X8 P172rn | AACAGGCTGATTCCCCTTTC | 53 |
| *ACVRL1* | ACVRL1-X10 M13fn2 | TGTCTTTCCTCTCGCTCTCC | 54 |
| *ACVRL1* | ACVRL1-X10 P172rn2 | ACCAGCACACACCACACTCAC | 55 |
| *BMPR2* | BMPR2-X1 f-M13 | GTGATACGGGCAGGATCAGT | 56 |
| *BMPR2* | BMPR2-X1 r-P172 | ATTTCCCTGGAAGGCATGG | 57 |
| *BMPR2* | BMPR2-X3 f-M13 | TAGCTTACACGTACTCTCAC | 58 |
| *BMPR2* | BMPR2-X3 r1-P172 | CATCACGCCTGGCTTCAAC | 59 |
| *BMPR2* | BMPR2-X6 f-M13 | CAGAGAGCTGTAGCATTCTG | 60 |
| *BMPR2* | BMPR2-X6 r-P172 | GCTGGAGTACGGCTGTACAAT | 61 |
| *BMPR2* | BMPR2-X7 Seqf | CGCATTTTTTCCTCTATATAG | 62 |
| *BMPR2* | BMPR2-X7 r-P172 | TTCCTGTTGTGAATTTTGAACC | 63 |
| *BMPR2* | BMPR2-X9F1-M13 | TTCAGGAAGGGCATTTTATAGG | 64 |
| *BMPR2* | BMPR2-X9R1-M13 | CATCCTGCTGCTAATAATGTTTC | 65 |
| *BMPR2* | BMPR2-X10 f-M13 | TTGGTATCAGAAATACCCCTGTT | 66 |
| *BMPR2* | BMPR2-X10 r-P172 | CAAACAACATTAAAACATATTCAGTCA | 67 |
| *BMPR2* | BMPR2-X11 Seqf | CACATGGTTTGACATGTACTTTG | 68 |
| *BMPR2* | BMPR2-X11 r-P172 | TCATTGAACTATTAGGCTGGTT | 69 |
| *BMPR2* | BMPR2-X12-3 f2 M13 | AGCAGAACCTTCCCAAGAGAC | 70 |
| *BMPR2* | BMPR2-X12-3 r2 P172 | TCTGAACATGGATTGCTGTTG | 71 |
| *BMPR2* | BMPR2-X12-4 f2 M13 | TGAGCATGAGCCTTTACTGAG | 72 |
| *BMPR2* | BMPR2-X12-4 r3 P172 | TAGTTATTTAAATGGCCCCA | 73 |
| *BMPR2* | BMPR2-X13 f-M13 | CACCCTCCTGAGACATTGGT | 74 |
| *BMPR2* | BMPR2-X13 r-P172 | CATCTTCTGCATGTTTAAATGA | 75 |
| *EIF2AK4* | EIF2AK4 X6 M13 | GTAATTAGCACATCCGTCACTGC | 76 |
| *EIF2AK4* | EIF2AK4 X6-35 P172 | GGAGTTTGCCCGATGTTTACC | 77 |
| *EIF2AK4* | EIF2AK4 X8-2 M13 | ACTTTCTGTTTAATGTGCAGGC | 78 |
| *EIF2AK4* | EIF2AK4 X8-4 P172 | CACCTTGGATGCTTACCTGC | 79 |
| *EIF2AK4* | EIF2AK4 X12 M13 | TGACTGTAGCTGTGGGACTC | 80 |
| *EIF2AK4* | EIF2AK4 X12-354 P172 | GTGTCACTTCGCCCTTGATC | 81 |
| *EIF2AK4* | EIF2AK4 X23 M13 | GACTTCAATGGTCCCAGTAGC | 82 |
| *EIF2AK4* | EIF2AK4 X23 P172 | TTCCTCCACCCCAGTAACAC | 83 |
| *EIF2AK4* | EIF2AK4 X38 M13 | AACCCCAGCAGTCTCCTAAC | 84 |
| *EIF2AK4* | EIF2AK4 X38 P172 | GGGACAGCTACTCCTAGGAC | 85 |
| *ENG* | ENG-X2 f | CCTCATAAGGTGGCTGTGATGATG | 86 |
| *ENG* | ENG-X2 r | CATCTGCCTTGGAGCTTCCTCT | 87 |
| *ENG* | ENG-X9 M13fn | TGGGTTGTGGTCAGTCCTTG | 88 |
| *ENG* | ENG-X9 P172rn | TGCTCTCCCAAACACACCTC | 89 |
| *ENG* | ENG-X13 M13fn | CTCCCAAAGGTGCCACATAC | 90 |
| *ENG* | ENG-X13 P172rn | CTAGGCTGCTATGGCTCTGG | 91 |
| *HRG* | HRG X1-66 M13 | CAGTGGCAGATCATAGCAAGG | 92 |
| *HRG* | HRGX1 P172 | AGCCATTCATTGTGCAAAGC | 93 |
| *SMAD1* | SMAD1-X3F | GCAGCAGGACAGGGATCAG | 94 |
| *SMAD1* | SMAD1-X3R | GTTGACCCAGCTTTTCCG | 95 |
| *SMAD4* | SMAD4 X3-43 M13 | CTCATGTGATCTATGCCCGTC | 96 |
| *SMAD4* | SMAD4 X3 P172 | ACTACAATACTCGGTTTTAGCAGTC | 97 |
| *SMAD6* | *SMAD6* X4 M13 | GTTGTGCGCCATATTTCCTGAG | 98 |
| *SMAD6* | SMAD6 X4-347 P172 | CGATCTTGCTGCGCGTTC | 99 |
| *SMAD7* | SMAD7 X1-232 M13 | CTTCTTCATGGTGTGCGGAG | 100 |
| *SMAD7* | SMAD7 X1-314 P172 | GCCGCTCCTTCAGTTTCTTG | 101 |
| *VCAN* | VCAN X3-117 M13 | GTTACAACACCAGTGAATTTCTCC | 102 |
| *VCAN* | VCAN X3 P172 | TTTTCCGCTCATCCAAACCC | 103 |
| *VCAN* | VCAN X7-735 M13 | CAGAAGACACCATCCACACTC | 104 |
| *VCAN* | VCAN X7-1938 P172 | TCTGTTCTCATCTCTGGTATTAGTG | 105 |
| *VCAN* | VCAN X7-2748 M13 | TGTTCCTCCCACTACCCTTG | 106 |
| *VCAN* | VCANX7 P172 | ACTCCAGTTATCTTCTAATAGCAGC | 107 |

Sensitivity and the specificity were estimated using *BMPR2, ALK1* and *ENG* genes fully sequenced with Sanger and NGS technique. Sensitivity is defined in our case as the percentage of mutations identified by Sanger (gold standard) which were also detected by NGS. By the set-up of NGS being followed by Sanger validation this two-step procedure has a specificity of 100%, since all NGS false positives are removed in the Sanger validation step.

Patients who showed no mutations within *BMPR2, ALK1* or *ENG* were additionally assessed with multiplex ligand-dependent probe amplification (MLPA, it P093-C1, MRC-Holland, Amsterdam, Netherlands) to detect large deletions or duplications encompassing exonic regions of *BMPR2, ALK1* and *ENG.*

### 1.7 Splice site mutation assessment

Suspected splice site mutations were investigated on RNA level. RNA was extracted following standard procedures. Complementary DNA was generated with a reverse transcriptase assay. Exons of interest were amplified in samples and controls with PCR and splice site alterations were visualised by agarose gel electrophoresis and Sanger sequencing; conditions are available upon request.

### 2. Results

### 2.1 Patient population

We included 37 patients and 5 first degree family members of affected individuals in the panel. Of the patients 19 suffered from IPAH, 8 had HPAH, one patient had PAH associated with congenital heart defects (CHD-APAH) and one patient APAH with pulmonary veno-occlusive disease. Eight patients had PAH and hereditary haemorrhagic telangiectasia (HHT) (Table 2). In the 5 family members no pulmonary hypertension or any other cardio-pulmonary disorder has been detected.

### 2.2 Clinical findings

Table 3 summarises the main clinical findings in the 37 PAH patients. Patients had severe PAH with impaired right ventricular function, lung function, lowered oxygen saturation and elevated NT-proBNP levels.

### 2.3 Results of PAH/CTEPH/PVOD/HHT-specific gene panel confirmed by direct Sanger sequencing

Figure 1 shows how the 42 subjects have been genetically assessed followed by bioinformatic processing. In 22 individuals of the 37 patients (59%) mutations in the *BMPR2, ALK1, EIF2AK4* or *ENG* genes (Table 4) and 12 heterozygous variants of uncertain significance (UVs, Table 5) in 6 further candidate genes were identified by NGS and validated by conventional Sanger sequencing. One patient carried a large heterozygous deletion of exon 6 to exon 7 in *BMPR2,* identified by MLPA (Table 4).

Two new heterozygous mutations in *EIF2AK4* gene were identified in a H/IPAH patient, respectively. The first *EIF2AK4* mutation led to an introduction of a stop codon (exon 8, c.933T>A p.(Y311*)). The second *EIF2AK4* mutation (exon 38, c.4892+1G>T) led to a loss of a splice site and subsequent RNA analysis confirmed the loss of exon 38. Twelve UVs were identified in the following PAH genes: *EIF2AK4, HRG, SMAD1, SMAD4, SMAD6, SMAD7* and *VCAN* (Table 5). Of the 12 UVs, four have not yet been identified in healthy individuals. However, these variants were only classified as "pathogenic" by 1-2 of the four *in silico* prediction programmes.

### 2.4 Sensitivity and specificity of the PAH-specific gene panel

The first of four runs of the panel diagnostic experienced technical errors during the sequencing by synthesis on the MiSeq; only 0.8 gigabases (Gb) were sequenced albeit with an average base call error rate ≤0.1% (≥Q30) of 91.2%. This drastically reduced the overall coverage leading to a lack of detection of 2 mutations previously identified with Sanger sequencing. These errors were clearly visible in quality parameters during sequencing and were addressed in the subsequent runs (run 2: 5.5 Gb, ≥Q30=90.5%; run 3: 5.8 Gb, ≥Q30=92.1%; run 4: 5.4 Gb, ≥Q30=90.6%). Hence, 100% sensitivity could be obtained in the following runs with an average coverage of approximately 800x. Nevertheless, 14 false positive mutations/variants were initially found with NGS panel which could not be confirmed by Sanger sequencing. These variants were either characterised by "short reads" of 25-36 bp (n=4), a very low heterozygosity of 10-15% (n=5) or other sequencing artefacts (n=5). Several of these artefacts were not confined to single patients and thus clearly identifiable as technical artefacts. Since in routine diagnostics all NGS mutations are verified with Sanger as a second step all false positives would be discovered. Therefore, a specificity of 100 is reached.

### 2.5 Comparison of sequencing and analysis methods

With the ultimate goal detecting new variants/mutations in PAH/CTEPH/PVOD/HHT related genes efficiently, the approach of NGS for a specific PAH/CTEPH/PVOD/HHT gene panel enabled highly sensitive, cost- and time-saving sequencing. Technical specifications of the commonly used Sanger sequencing and the new PAH/CTEPH/PVOD/HHT-specific gene panel diagnostic with NGS were compared in Table.

The panel diagnostic in the pilot study allowed sequencing all exons in 29 genes at the same time instead of only one direction of one exon per reaction by Sanger. Not only each exon has to be sequenced separately with Sanger sequencing but also each patient. Meanwhile, the panel diagnostic allows assessing up to 96 patients at the same time albeit keeping the data storage requirements at a moderate level.

The Sanger technique requires approximately 11 h assessment time/per patient for the 3 major PAH/CTEPH/HHT genes *BMPR2, ALK1* and *ENG.* In contrast, the new panel technique in our diagnostic setting allowed the assessment of 29 genes within 5 h assessment time/per patient plus the time required for confirmatory Sanger sequencing. This huge time advantage was enabled by processing several patient samples and PAH/CTEPH/PVOD/HHT genes simultaneously in the laboratory (48 h sample processing in the laboratory for 12 samples at once, see Table 6). While data generation is faster than Sanger sequencing, data analysis and bioinformatics processing is prolonged as any possible pathogenicity of rare variants has to be investigated by *in silico* prediction programmes and literature research. Nevertheless, once a data base has been established classifying the effect of variants data analysis can be accelerated.

When large deletions and insertions are identified with the PAH/CTEPH/PVOD/HHT-specific gene panel based on NGS, it is preferred to confirm such findings with a second method such as MLPA or quantitative PCR. The PAH/CTEPH/PVOD/HHT-specific gene panel is not limited to the sequence data analysis regarding mutations but also enables the detection of large deletions or insertions in the routine diagnostic setting.

**Table 1: PAH/CTEPH causal genes and candidate genes (of the pilot study)**

| **Gene name** | **ENSEMBL Gene ID** | **Gene size (bp)** | **Exon number** | **Transcribed exons** |
|---|---|---|---|---|
| *ACVR1* | ENSG00000115170 | 139418 | 12 | 4-12 |
| ***ACVRL1*** | | 16453 | 10 | 2-10 |
| *(ALK1)* | ENSG00000139567 | | | |
| *BMP2* | ENSG00000125845 | 12616 | 3 | 2-3 |
| *BMPR1A* | ENSG00000107779 | 176188 | 13 | 3-13 |
| ***BMPR1B*** | ENSG00000138696 | 400480 | 13 | 4-13 |
| ***BMPR2*** | ENSG00000204217 | 190815 | 13 | 1-13 |
| *CAV1* | ENSG00000105974 | 36399 | 3 | 1-3 |
| *CREB* | ENSG00000118260 | 73694 | 9 | 3-9 |
| ***EIF2AK4*** | ENSG00000128829 | 101445 | 39 | 1-39 |
| *ENG* | ENSG00000106991 | 39744 | 15 | 1-15 |
| *HRG* | ENSG00000113905 | 18024 | 7 | 1-7 |
| *ID1* | ENSG00000125968 | 1232 | 2 | 1-2 |
| *ID2* | ENSG00000115738 | 5608 | 5 | 2-3 |
| *ID3* | ENSG00000117318 | 1876 | 3 | 1-2 |
| *ID4* | ENSG00000172201 | 3298 | 3 | 1-2 |
| *IL6* | ENSG00000136244 | 6118 | 6 | 2-6 |
| ***KCNA5*** | ENSG00000130037 | 1842 | 6 | 1 |
| ***KCNK3*** | ENSG00000171303 | 40730 | 2 | 1-2 |
| ***SMAD1*** | ENSG00000170365 | 76885 | 7 | 2-7 |
| ***SMAD4*** | ENSG00000141646 | 56651 | 12 | 2-12 |
| *SMAD5* | ENSG00000113658 | 55902 | 8 | 3-8 |
| *SMAD6* | ENSG00000137834 | 80620 | 4 | 1-4 |
| *SMAD7* | ENSG00000101665 | 30858 | 4 | 1-4 |
| ***SMAD9*** | ENSG00000120693 | 75934 | 7 | 2-7 |
| *SOD2* | ENSG00000112096 | 93472 | 6 | 1-5 |
| ***TOPBP1*** | ENSG00000163781 | 63718 | 28 | 2-28 |
| *VCAN* | ENSG00000038427 | 110838 | 15 | 2-15 |
| *VHL* | ENSG00000134086 | 11212 | 3 | 1-3 |
| *ZFYVE16* | ENSG00000039319 | 71337 | 19 | 3-19 |

Bold genes indicate 12 of the 14 known PAH genes.

**Table 2: Characteristics of study population**

| **Characteristics of study population** | | | |
|---|---|---|---|
| Gender, n (%) female | 35 | | (83%) |
| Age (years) | 48 | ± | 13 |

| Disease | | | |
|---|---|---|---|
| IPAH | 19 | | (45%) |
| HPAH | 8 | | (19%) |
| CHD-APAH | 1 | | (2%) |
| PVOD-AP AH | 1 | | (2%) |
| PAH and HHT | 8 | | (19%) |
| Family member of P AH patient | 5 | | (12%) |

| | | | |
|---|---|---|---|
| Values are given as n (%) or mean ± standard deviation IPAH: idiopathic pulmonary arterial hypertension, HPAH: hereditary pulmonary arterial hypertension, CHD-APAH: congenital heart defect with associated PAH, PVOD: pulmonary veno-occlusive disease, HHT: hereditary haemorrhagic telangiectasia | | | |

**Table 3: Clinical characteristics of the 37 PAH patients**

| **Characteristic*** | **Mean ± Standard deviation** | | |
|---|---|---|---|
| Age at first diagnosis of PAH [years], | 50 | ± | 13 |
| n=37 | | | |
| Age at first diagnosis of HHT [years], | 9 | ± | 3 |
| n=8 | | | |

| **6-minute walking test** | | | |
|---|---|---|---|
| 6MWD [m] | 415 | ± | 53 |
| Borg Scale out of 20 | 13 | ± | 2 |
| SaO₂ after 6MWD [%] | 92 | ± | 2 |

| **Lung function** | | | |
|---|---|---|---|
| Vital capacity max [%] | 79 | ± | 17 |
| Forced vital capacity [%] | 88 | ± | 9 |
| FEV1 [%] | 72 | ± | 17 |
| Peak expiratory flow [%] | 83 | ± | 11 |
| SaO₂ at rest [%] | 96 | ± | 1 |

| **Echocardiography at rest and during exercise** | | | |
|---|---|---|---|
| Right atrial area [cm²] | 23 | ± | 8 |
| Right ventricular area [cm²] | 22 | ± | 4 |
| Vena cava inferior [cm] | 1.9 | ± | 0.1 |
| sPAP at rest [mmHg] | 64 | ± | 19 |
| TAPSE [cm] | 2.2 | ± | 0.5 |
| TDI-s [cm/s] | 12 | ± | 3 |
| Left ventricular eccentricity index | 1.3 | ± | 0.3 |
| Workload during exercise [Watt] | 79 | ± | 23 |
| sPAP during exercise [mmHg] | 97 | ± | 23 |

| **Right heart catheter** | | | |
|---|---|---|---|
| mPAP [mmHg] | 53 | ± | 11 |
| PAWP [mmHg] | 10 | ± | 4 |
| Cardiac output [1/min] | 4.3 | ± | 1.4 |
| Cardiac index [1/min/m²] | 2.5 | ± | 0.8 |
| PVR [dynes] | 1045 | ± | 517 |

| **Laboratory** | | | |
|---|---|---|---|
| NT-proBNP [pg/ml] | 1800 | ± | 2289 |

| | | | |
|---|---|---|---|
| *not all measurements were obtained from each patient data are given as mean ± standard deviation SaO₂: arterial oxygen saturation, 6MWD: 6-minute walking distance FEV1: Forced expiratory volume 1st second, sPAP: systolic pulmonary arterial pressure, TAPSE: Tricuspid annular plane systolic excursion mPAP: Mean pulmonary artery pressure, PAWP: Pulmonary arterial wedge pressure, PVR: Pulmonary vascular resistance | | | |

**Table 4: Variants/mutations detected with Sanger and NGS-gene panel**

| **Gene** | **Exon** | **Variant name** | **Impact** | **Sanger** | **Panel** |
|---|---|---|---|---|---|
| *ACVRL1* | 3 | c.128_132delGGCCT | deletion | yes | yes |
| | | p.(G43D)fs*124 | | | |
| *ACVRL1* | 5 | c.595G>C p.(A199P) | missense | yes | yes |
| *ACVRL1* | 6 | c.662G>A p.(W221 *) | nonsense | yes | yes |
| *ACVKL1* | 7 | c.788A>G p.(D263G) | missense | yes | yes |
| *ACVRL1* | 7 | c.950T>C p.(I317T) | missense | yes | yes |
| *ACVRL1* | 8 | c.1055C>A p.(A352D) | missense | yes | yes |
| *ACVRL1* | 8 | c.1231C>T p.(R411W) | missense | yes | yes |
| *ACVRL1* | 10 | c.1451G>A p.(R484Q) | missense | yes | yes |
| *BMPR2* | 1 | c.76+1G>T | intronic | yes | yes |
| *BMPR2* | 3 | c.377A>G, p.(N126S) | missense | yes | * |
| *BMPR2* | 3 | c.418+5G>A | intronic | yes | yes |
| *BMPR2* | 6 | c.631C>T p.(R211*) | nonsense | yes | yes |
| *BMPR2* | 6 | c.659dupG p.(G220fs*4) | duplication | yes | yes |
| *BMPR2* | 7 | c.967+5G>A | intronic | yes | yes |
| *BMPR2* | 6-7 | deletion exon 6-7** | large deletion | no | no |
| *BMPR2* | 9 | c.1129-1_1129dupGG p.(V377Gfs*13) | duplication | yes | yes |
| *BMPR2* | 10 | c.1277-9A>G | splice defect | yes | * |
| *BMPR2* | 10 | c.1297C>T p.(Q433*) | nonsense | yes | yes |
| *BMPR2* | 11 | c.1453G>A p.(D485N) | missense | yes | yes |
| *BMPR2* | 12 | c.2308delC p.(R770Gfs*2) | deletion | yes | yes |
| *BMPR2* | 12 | c.2695C>T p.(R899*) | nonsense | yes | yes |
| *BMPR2* | 13 | c.3062_3064delAAG p.(E1021..1022G) | deletion | yes | yes |
| *EIF2AK4* | 8 | c.933T>A p.(Y311*) | nonsense | yes | yes |
| *EIF2AK4* | 38 | c.4892+1G>T | splice defect | yes | yes |
| *ENG* | 2 | c.90T>G p.(C30W) | missense | yes | yes |
| *ENG* | 9 | c.1268A>G p.(N423S) | missense | yes | yes |
| *ENG* | 13 | c.1741_1742dupTCTG p.(G581fs) | duplication | yes | yes |

| | | | | | |
|---|---|---|---|---|---|
| *Technical errors during the run were evident during the data generation. These samples would be repeated during diagnostics due to low quality values. **This large deletion was detected with MLPA and was not visible with Sanger nor NGS | | | | | |

**Table 5: New variants of unknown significance detected by NGS and confirmed with Sanger sequencing**

| Gene | Exon | Variant name | Impact | MutationTaster | SIFT | PolyPhen2 | Align GVGD | % in ExAC |
|---|---|---|---|---|---|---|---|---|
| *EIF2AK4* | 6 | c.627A>C p.(Q209H) | missense | benign | benign | pathogenic | benign | 0.0041% |
| *EIF2AK4* | 12 | c.1819-16C>G | intronic | benign | NA | NA | NA | - |
| *EIF2AK4* | 23 | c.3329T>Gp.(M1110R) | missense | pathogenic | benign | pathogenic | benign | - |
| *HRG* | 1 | c.167C>T p.(A56V) | missense | pathogenic | pathogenic | pathogenic | pathogenic | 0.0568% |
| *SAMD1* | 3 | c.549G>A p.(P183P) | synonymous | pathogenic | NA | NA | NA | 0.0016% |
| *SMAD4* | 3 | c.354G>A p.(A118A) | synonymous | benign | NA | NA | NA | 0.0016% |
| *SMAD6* | 4 | c.173C>T p.(A58V) | missense | benign | pathogenic | benign | benign | - |
| *SMAD7* | 1 | c.115G>A p.(G39R) | missense | pathogenic | pathogenic | pathogenic | benign | 0.028% |
| *SMAD7* | 3 | c.709G>A p.(G237R) | missense | pathogenic | pathogenic | benign | uncertain | - |
| *VCAN* | 3 | c.301G>T p.(V101L) | missense | pathogenic | pathogenic | pathogenic | benign | 0.0016% |
| *VCAN* | 7 | c.1045A>G p.(K349E) | missense | benign | pathogenic | pathogenic | pathogenic | 3.106% |
| *VCAN* | 7 | c.4003+16C>G | intronic | pathogenic | NA | NA | NA | 0.1087% |

| | | | | | | | | |
|---|---|---|---|---|---|---|---|---|
| ExAC: Exome Aggregation Consortium | | | | | | | | |

**Table 6: Comparison of common Sanger sequencing with the PAH-specific gene panel in our diagnostic setting**

| **Characteristic** | **Sanger Sequencing** (3 genes) | **PAH-specific gene panel by NGS** (29 genes) |
|---|---|---|
| Genes | *BMPR2, ALK1, ENG* | *BMPR2, ALK1, ENG, EIF2AK4, KCNK3, CAV1,* ... |

| Time expense | | |
|---|---|---|
| Preparation time/gene | 8 hours | 24 hours |
| Sequencing time/gene | 2 hours | 24 hours |
| Analysis time of data/patient | 1 hour | 3 hours |
| Total time effort | 11 hours | 51 hours |
| Maximum patients per run | 1 patient | 12 patients |
| Exons per patient per reaction | 1 exon | All exons of 29 genes |
| Total time per patient | 11 hours | 5 hours* |

| Data storage | | |
|---|---|---|
| Implementation and handling | Easy | Moderate |

| | | |
|---|---|---|
| *these data refer to average values per patient considering a joined preparation of 12 samples | | |

### EXAMPLE 2 EIF2AK4 mutation as "second hit" in hereditary pulmonary arterial hypertension.

### 1. Materials and Methods

### 1.1 Subjects and clinical characterisation

Members of a family with autosomal dominantly inherited HPAH were clinically and genetically assessed. All living genetically related family members were invited to participate in a clinical and genetic evaluation. After written informed consent was obtained family members underwent clinical assessment and genetic counseling. A three generation pedigree was drawn including nine family members of the index patient. EDTA-blood was taken for genetic analysis.

Clinical procedures consisted of recording the family and medical history, physical examination, laboratory parameters including N-type pro brain natriuretic peptide (NT-proBNP), 12-lead electrocardiogram, lung function test, arterial blood gases, 6-minute walking distance, echocardiography, stress-Dopplerechocardiography and cardiopulmonary exercise testing as described previously (Hinderhofer *et al.,* 2014). High resolution computer tomography of the lung was conducted to exclude pulmonary veno-occlusive disease. Left heart catheterisation was performed in all patients with suspected left heart diseases and when clinically indicated. Manifest HPAH was diagnosed according to the current guidelines (Galiè *et al.,* 2016). Right heart catheterisation was performed in the living HPAH patients to confirm diagnosis and for follow-up.

### 1.2 Genetic assessment

Genomic DNA was isolated from peripheral blood using a salting out procedure (Miller *et al.,* 1988) (Autopure, LGC, Germany). Sanger sequencing for *BMPR2* (ENST00000374580) was conducted in the index patient using Big Dye Terminator V1.1 cycle sequencing kit and ABI 3130x1 genetic analyzer (ThermoFisher Scientific, USA). Duplications and deletions were screened by multiplex ligation-dependent probe amplification (MLPA, kit P093-C2, MRC-Holland, the Netherlands).

A new PAH gene panel diagnostic based on next-generation sequencing was designed to analyse second hit mutations in 11 PAH (*ACVRL1* or *ALK1, BMPR1B, BMPR2, CAV1, EIF2AK4, ENG, KCNA5, KCNK3, SMAD1, SMAD4, SMAD9*) and 20 further candidate genes in the index patient. DNA was enriched with a customised SureDesign panel (Agilent Technologies, USA) and sequenced on the MiSeq (Illumina, USA). Exonic regions and exon-intron boundaries were analysed with SeqPilot 4.1.2 (JSI medical systems GmbH, Germany). Variants were characterised following the recommendations of the Human Genome Variation Society (HGVS version 2.15.11) (den Dunnen *et al.,* 2016). Non-synonymous missense variants with a population frequency <5% were assessed regarding their evolutionary conservation, location within functional gene domains and functional consequence using four *in silico* prediction programs: MutationTaster, SIFT, Align GVGD and PolyPhen2 implemented in Alamut Visual 2.7.1 (interactive biosoftware, France). Variants were confirmed by Sanger sequencing in the index patient and assessed in family members to clarify mutation status. Any variants disrupting gene function were considered mutations.

### 1.3 Functional assessment of mutations

RNA was isolated from EDTA blood using standard procedures. Copy DNA (cDNA) was generated with a reverse transcriptase reaction adding hexamers for 10 min at 65°C, a cDNA-Mix (Invitrogen, USA) for 2 hours at 37°C and a final reaction for 10 min at 65°C.

A PCR was designed to assess the cDNA corresponding to the messenger RNA of *EIF2AK4* (ENSG00000128829; SEQ ID NO. 5) The PCR product span the exon 38 using a forward primer annealing in exon 36 (5' GACCTCCCTTGCCAACTTAC 3'; SEQ ID NO. 108) and a reverse primer annealing in exon 39 (5' AGAT TCTGTAGTAGTCATCTCTATAGC 3'; SEQ ID NO. 109). The expected size of the intact PCR product was 266 bp and the one of the PCR product skipping exon 38 was 147 bp. cDNA was denatured for 5 min at 95°C, and subsequently amplified in 35 cycles (1 min at 95°C, 1 min at 56.5°C, 1.5 min at 72°C; final elongation 10 min at 72°C). While several transcripts exist for *EIF2AK4* the considered isoform is the most common one in humans since it is referred to as reference sequence by NCBI RefSeq. NCBI RefSeq is a database which we used as reference standard for reporting the location of medically important variations. PCR products were sequenced with Sanger sequencing to identify the altered base pair sequence of the messenger RNA.

### 1.4 Statistical analyses

Significance of co-occurance of *BMPR2* and *EIF2AK4* was calculated by using an estimated heterozygote frequency of *EIFAK4* mutations retrieved from the Exome Aggregation Consortium (ExAC) database (Lek *et al.,* 2016) and taking into account the number of genes on the panel by correcting for multiplicity. A p-value lower than 5% was considered statistically significant.

### 2. Results

### 2.1 Clinical characterisation

Clinical parameters are presented in Table 7. PAH was excluded in healthy family members by regular clinical assessments including physical examination, lung function tests, ECG, and in particular by echocardiography, stress-Doppler-echocardiography, spiroergometry and NT-proBNP values. Diagnosis of PAH was confirmed by right heart catheterisation in patients II:4, III:2 and III:3. The disease was very severe in all three patients but could be stabilised with medication in patients II:4, and III:2. Patient III:3 showed a rapid progression and received a lung transplantation 1.5 years after diagnosis. She died only one year after transplantation due to a rejection of the donor organ. Patient II:4 and III:2 were under dual therapy at the time of this writing. Stable haemodynamic parameters were observed in the latest catheter of patient II:4 with a mean pulmonary artery pressure (mPAP) of 46 mmHg, pulmonary arterial wedge pressure (PAWP) of 12 mmHg, cardiac output 4.6 l/min, cardiac index 2.3 l/min/m² and pulmonary vascular resistance of 591 dynes. Patient III:2 has improved under therapy to a mPAP of 55 mmHg, PAWP of 14 mmHg, cardiac output 6.0 l/min, cardiac index 2.9 l/min/m² and pulmonary vascular resistance of 547 dynes. Pulmonary veno-occlusive disease (PVOD) was excluded in the patients on three grounds. Firstly, high resolution computer tomography showed no morphological changes typical for PVOD. Secondly, diffusion capacity of the lung for carbon monoxide (DLCO) values were greater than 50% predicted. Values around 50% are often seen in PVOD (Montani *et al.,* 2008). Thirdly, patients II:4 and III:2 have received PAH medication for eight years and were stable under dual therapy. Whereas in PVOD a worsening of symptoms is often observed, once PAH medication has been given.

**Table 7: Clinical parameters**

| **Parameter** | **II:4** | **III:2** | **III:4** |
|---|---|---|---|
| Age at diagnosis | 49 | 29 | - |
| mPAP [mmHg]¹ | 55 | 63 | - |
| PAWP [mmHg]² | 13 | 14 | - |
| CO [l/min] | 6.1 | 5.1 | - |
| CI [l/min/m²] | 3.2 | 2.4 | - |
| PVR dynes | 551 | 736 | - |
| SaO₂ % | 97 | 98 | 96 |
| sPAP [mmHg] at rest³ | 94 | 47 | 20 |
| sPAP [mmHg] during exercise³ | 100 | 95 | 29 |
| Peak VO₂ [ml/min/kg] | 18 | 17 | 24 |
| RV area [cm²] | 18 | 27 | 15 |
| TAPSE [cm] | 3.0 | 2.4 | 2.3 |
| DLCO predicted [%] | 77 | 60 | 91 |
| NT-proBNP | 89 | 58 | 31 |
| 6-MWD | 480 | 560 | - |
| Medication | Silenafil, Macitentan | Sildenafil, Macitentan | - |

| | | | |
|---|---|---|---|
| ¹mPAP ≥25 mmHg characterises pulmonary hypertension ²PAWP >15 mmHg together with mPAP ≥25 mmHg characterises post-capillary pulmonary hypertension due to left heart disease; PAWP ≤15 mmHg together with mPAP ≥25 mmHg characterizes pre-capillary PH ³sPAP >40 mmHg at rest and sPAP >45 mmHg at low workloads is considered here as abnormal and exercise induced pulmonary hypertension, respectively (Nagel *et al.,* 2015). However, cut-offs are not clearly defined in current guidelines. mPAP, (sPAP): mean (systolic) pulmonary arterial pressure, PAWP: pulmonary arterial wedge pressure, CO: cardiac output, CI: cardiac index, SaO₂: oxygen saturation, VO₂: oxygen uptake, PVR: pulmonary vascular resistance, RV: right ventricular, TAPSE: tricuspid annular plane systolic excursion, DLCO: diffusion capacity of the lung for carbon monoxide, NT-proBNP: N-type pro brain natriuretic peptide, 6-MWD: 6 minute walking distance | | | |

### 2.2 Genetic findings

All living family members with manifest HPAH (II:4, III:2) carried a heterozygous mutations in *BMPR2* and *EIF2AK4.* In contrast, two non-diseased family members (III:1 and III:4) carried only the *BMPR2* mutation and no mutation in *EIF2AK4.* None of these family members developed manifest HPAH at of the time of this writing.

The first familial mutation lay within exon 12 of the *BMPR2* gene (c.2308delC, p.(Arg770Glyfs*2)) and led to the deletion of a cytosine resulting presumably in a premature stop codon two amino acids downstream. The second mutation was located one base pair behind the end of exon 38 of the gene *EIF2AK4* (c.4892+1G>T) resulting in the loss of the complete exon 38 (Figure 2). Due to this exon loss sequencing results suggested a frame shift and subsequent introduction of a premature stop codon in exon 39 at amino acid position 1599 instead of position 1650 in the regular protein; hence, a disrupted protein after the end of exon 37 is predicted.

While DNA was only available from five family members the pedigree analysis revealed two further obligate carriers of the *BMPR2* mutation. The grandfather (I:1) and the aunt (II:2) of the index patient must have been carriers for the cousin (III:1) to harbour the mutation. At the same time, the *EIF2AK4* mutation was most likely also present in the grandfather (I:1), who had died aged 49 years due to liver cirrhosis. Since the mother of the index patient (II:4) only developed manifest PAH at the age of 49 years, it is likely that the grandfather would have developed PAH due to the inferred presence of both mutations at a later stage in his life or had already developed the disease but had not been diagnosed until his death. Alternatively, the *EIF2AK4* mutation might have arisen *de novo* in II:4 or as a germ line mosaicism in either grandparent of the index patient.

In HPAH we expect a *BMPR2* mutation with a probability of 85% (Machado *et al.,* 2015; Pfarr *et al.,* 2011). Under the null hypothesis stating that the mutation in *EIF2AK4* does not contribute to disease manifestation but instead represents a random event for the proband, the chance to observe the association of the two mutations has the probability of 0.00046. Taking the number of genes on the panel into account this association of both mutations under the null hypothesis reveals p=0.00046*30=0.014, which is significant below the 5% level. Hence, the co-occurrence of both mutations in affected family members is a significant association. Moreover, *EIF2AK4* co-segregates with disease conditioned on all *BMPR2* positive family members supporting the hypothesis of a second hit model, in which the diplotype of mutations in both genes has a high penetrance for PAH.

### EXAMPLE 3 Identification KLF-2 mutation in heritable pulmonary arterial hypertension.

### 1. Materials

### 1.1 Subjects and clinical characterisation

Members of a HPAH family were genetically and clinically assessed after providing their written informed consent for genetic and clinical analysis. The study was approved by the Ethics Committee at Heidelberg University and carried out in accordance with the Declaration of Helsinki (2013) of the World Medical Association. Diagnosis of PAH was confirmed by right heart catheterisation in all affected family members. Family members with no symptoms or signs of PAH were assessed by obtaining medical history, physical examination, 12-lead ECG, lung function test, chest X-ray, echocardiography at rest and during exercise and laboratory assessment as described previously (Ehlken *et al.,* 2016).

### 1.2 Sanger sequencing and MLPA

DNA was extracted from peripheral blood using standardised procedures (Autogene Qiagen, Germany). Sanger sequencing using Big Dye Terminator V1.1 cycle sequencing kit and ABI 3130x1 genetic analyser and multiplex ligation-dependent probe amplification (MLPA, kit P093-C2, MRC-Holland, the Netherlands) was conducted for the index patient for the genes *BMPR2* (ENSG00000204217) [SEQ ID NO. 3], *ACVRL1* (ENSG00000139567) [SEQ ID NO. 1] and *ENG* (ENSG00000106991) [SEQ ID NO. 6].

### 1.3 Next-generation sequencing and variant analysis

Next-generation sequencing (NGS) was carried out to investigate potential mutations in PAH and candidate genes as described previously (Song *et al.,* 2016). All known PAH genes (*ACVRL1, BMPR1B, BMPR2, CAV1, EIF2AK4, ENG, KCNA5, KCNK3, SMAD1, SMAD4, SMAD9* and *TOPBP1*) and further candidate genes from the BMPR2 pathway and genes which were shown to have a PAH relevance in animal models such as *KLF2, KLF4* and *KLF5* were included in a PAH-specific gene panel. DNA was enriched with a customised SureDesign panel (Agilent Technologies), sequenced on the MiSeq and analysed with SeqPilot 4.1.2 as described previously (Song *et al.,* 2016). Variants in exonic regions and exon-intron boundaries were characterised following the recommendations of the Human Genome Variation Society (HGVS, http://www.hgvs.org;version 2.15.11) (den Dunnen *et al.,* 2016). Non-synonymous missense variants with a population frequency of less than 5% were assessed regarding their evolutionary conservation, location within functional gene domains and their functional consequence using four *in silico* prediction programmes: MutationTaster, SIFT, Align GVGD and PolyPhen2 implemented in Alamut Visual 2.7.1. Furthermore, in order to determine gene function, a systematic literature search was performed to investigate mutations, their functions within the cell and their involvement in the *BMPR2* pathway, which is affected in most HPAH families (Machado *et al.,* 2015). Any variants disrupting the function of a gene were considered mutations.

The COSMIC data base (Forbes *et al.,* 2015) for somatic mutations and HGMD for germline mutations, were used respectively together with a systematic literature search in PubMed to list previously reported mutations. Cellular and molecular functions of affected genes relevant to phenotypes observed in PAH were also investigated by a broad literature search.

Potential mutations were confirmed with Sanger sequencing guaranteeing a 100% specificity. Family members of the index patient were assessed with Sanger sequencing to clarify mutation status.

### 1.4 Statistical analyses

Thirteen unrelated IPAH/ HPAH patients without mutations in the genes *BMPR2, ENG* and *ACVRL1* were screened for the presence of rare mutations using NGS assessing all exons and exon-intron boundaries. The Fisher's exact test was used to compare mutations in our sample to 126,216 controls (Lek *et al.,* 2016) calculated with BIAS v.11.02.

### 2. Results

### 2.1 Clinical characteristics

Three family members of the HPAH family were diagnosed with invasively confirmed manifest PAH. The father (II:4) of the index patient (III:3) had died one year after diagnosis of severe and rapidly progressive HPAH and right heart failure aged 32 years. His two daughters (III:2 and III:3) were diagnosed with HPAH in 1993 and suffered from severe and rapidly progressive disease unresponsive to medical therapy (Table 8). They underwent lung and heart/lung transplantation, respectively, shortly after diagnosis both at the age of 25 years. Both of them are still alive and are doing well as of the time of this writing having been clinically followed for more than 20 years after transplantation. Neither of the sisters had any children. The brother (III:1) and further family members remained unaffected up to the present day with a clinical follow-up of >20 years as well.

**Table 8 Clinical characterization of affected family members**

| **Parameter** | **III:2** | **III:3** |
|---|---|---|
| Age at diagnosis | 25 | 25 |
| Age at transplantation | 28 | 27 |
| Heart rate [min⁻¹] | 85 | 92 |
| Blood pressure [mmHg] | 115/80 | 110/80 |

| Lung function | | |
|---|---|---|
| FEV1 % | 94 | 60 |
| Total resistance [kPa*s/l] | 0.29 | 0.19 |
| SaO₂ % | 98 | 97 |

| Blood gases | | |
|---|---|---|
| PO₂ [mmHg] | 89 | 91 |
| PCO₂ [mmHg] | 23 | 27 |

| Radionuclide ventriculography | | |
|---|---|---|
| LVEF % | 60 | 60 |
| RVEF % | 34 | 32 |

| Echocardiography | | |
|---|---|---|
| sPAP [mmHg] | 67 | 110 |

| Right heart catheter | | |
|---|---|---|
| mPAP [mmHg] | 48 | 72 |
| PAWP [mmHg] | 10 | 10 |
| PVR [dynes*sec*cm⁻⁵] | 880 | 768 |
| CO [l/min] | 3.9 | 5.9 |
| CI [l/min/kg] | 2.6 | 3.7 |

| | | |
|---|---|---|
| FEV1: forced expiratory volume in the first second, SaO₂: arterial oxygen saturation, PO₂: partial pressure of oxygen, PCO₂: partial pressure of carbon dioxide, LVEF: left ventricular ejection fraction, RVEF: right ventricular ejection fraction, sPAP: systolic pulmonary artery pressure, mPAP: mean pulmonary artery pressure, PAWP: pulmonary artery wedge pressure, PVR: pulmonary vascular resistance, CO: cardiac output, CI: cardiac index | | |

### 2.2 Genetic analyses

In the genetic analysis of the two affected sisters, no disease-causing mutation, deletion or duplication was found in the known and previously published PAH genes (Table 9). By including new candidate genes in the NGS approach, we identified a new, germline heterozygous missense mutation in exon 2, c.862C>T p.H288Y in the *Krüppel-like factor 2* (*KLF2*) gene on chromosome 19 (ENSG00000127528) [SEQ ID NO. 10] in both affected sisters (Table 9).

**Table 9 Genetic analysis**

| **Genetic analysis** | | **Genes included** | **Results** |
|---|---|---|---|
| Sanger sequencing /MLPA | Major PAH genes | *BMPR2, ACVRL1, ENG* | Negative |
| Next-generation sequencing | Further known candidate genes | *BMPR1B, CAV1, EIF2AK4, KCNA5, KCNK3, SMAD1, SMAD4, SMAD9, TOPBP1* | Negative |
| Next-generation sequencing | New candidate genes | *ACVR1, BMP2, BMPR1A, CREB, HRG, ID1, ID2, ID3, ID4, IL6, KLF2, KLF4, KLF5, SMAD5, SMAD6, SMAD7, SOD2, VCAN, VHL, ZFYVE16* | Germline heterozygous mutation in exon 2, c.862C>T p.H288Y in *KLF2,* on chromosome 19p13.11 |

No DNA was available from the father who died early due to HPAH. Since the mutation was absent in the healthy mother (II:5) we infer its presence in the deceased father (II:4). We screened all first degree related family members of the father for the presence of the mutation in *KLF2* with Sanger sequencing. The mutation was absent in all paternal siblings (II:1-II:3). None of them nor the grand-parents (I:1, I:2) of the index-patient developed PAH.

This germline mutation was absent in exomes of >120,000 control subjects assessed by the Exome Aggregation Consortium and the genome Aggregation Database (Lek *et al.,* 2016) .

Similarly, *KLF2* was entirely absent from the germline mutation data bases HGMD and ClinVar. The Fisher's exact test showed that the absence of the mutation in 126,216 controls compared to our sample (1/14, 2-sided p-value=0.0001) demonstrated a significant association of the *KFL2* variant with PAH. The effect of the mutation was classified as pathogenic by four *in silico* prediction programmes (respective scores: MutationTaster: 1, SIFT: 0, PolyPhen2: 0.927, Align GVGD: C65). The mutation led to the substitution of a highly conserved nucleotide and subsequently the exchange of a highly conserved amino acid within a zinc finger protein domain. This domain is characterised by two cysteine and two histidine residues (2Cys-2His) forming two beta sheets and one alpha helix.

The mutation might have occurred *de novo* in the father, who could not be analysed, or was inherited by one of the grandparent presenting with reduced penetrance. Under both assumptions *KLF2* co-segregates with the disease in this family based on the genetically analysed family members. So far we could not identify further *KLF2* mutations in 13 other unrelated IPAH/ HPAH patients without mutations in *BMPR2, ENG* or *ACVRL1.*

### 2.3 Involvement of the KLF2 gene in PAH pathways:

*KLF2* is highly important for lung functioning, represented by its previous name "Lung KLF" and its high expression in the adult mouse lung (Anderson *et al.,* 1995). We summarised the diverse array of cellular processes regulated by KLF2 such as cell differentiation, migration, coagulation and tissue development (Kumar *et al.,* 2005), which are all involved in PAH pathogenesis. Studies implicated KLF2 as a key transcriptional regulator of endothelial pro-inflammatory activation (Bhattacharya *et al.,* 2005; SenBanerjee *et al.,* 2004). KLF2 also inhibited endothelial activation and proliferation (Bhattacharya *et al.,* 2005) as well as the expression of endothelin-1, adrenomedullin and angiotensin converting enzyme, all of which increase vascular contractile tone (Dekker *et al.,* 2005).

Mice with homozygous germline deletions of *KLF2* die around day 14 during development due to heart failure preceded by a high cardiac output state (Lee *et al.,* 2006). In contrast, heterozygous mice are viable and fertile (Yeo *et al.,* 2014). *KLF2* overexpression in a PH model of hypoxic rats was able to reduce right ventricular systolic pressure (RVSP) while its knock down increased RVSP and reduced the expression of the vasodilator endothelial nitric oxide synthase (eNOS) in normoxia (Dungey *et al.,* 2011).

### REFERENCES

Anderson KP, Kern CB, Crable SC, and Lingrel JB. 1995. Isolation of a gene encoding a functional zinc finger protein homologous to erythroid Kruppel-like factor: identification of a new multigene family. Mol Cell Biol 15(11):5957-5965.
Austin ED, Ma L, LeDuc C, Berman Rosenzweig E, Borczuk A, Phillips JA, 3rd, et al. Whole exome sequencing to identify a novel gene (caveolin-1) associated with human pulmonary arterial hypertension. Circ Cardiovasc Genet. 2012;5(3):336-43.
Barrios-Rodiles M, Brown KR, Ozdamar B, Bose R, Liu Z, Donovan RS, Shinjo F, Liu Y, Dembowy J, Taylor IW et al.. 2005. High-throughput mapping of a dynamic signaling network in mammalian cells. Science 307(5715):1621-1625.
Berlanga JJ, Santoyo J, and De Haro C. 1999. Characterization of a mammalian homolog of the GCN2 eukaryotic initiation factor 2alpha kinase. Eur J Biochem 265(2):754-762.
Best DH, Sumner KL, Austin ED, Chung WK, Brown LM, Borczuk AC, et al. EIF2AK4 mutations in pulmonary capillary hemangiomatosis. Chest. 2014;145(2):231-6.
Bhattacharya R, Senbanerjee S, Lin Z, Mir S, Hamik A, Wang P, Mukherjee P, Mukhopadhyay D, and Jain MK. 2005. Inhibition of vascular permeability factor/vascular endothelial growth factor-mediated angiogenesis by the Kruppel-like factor KLF2. J Biol Chem 280(32):28848-28851.
Chandra SM, Razavi H, Kim J, Agrawal R, Kundu RK, de Jesus Perez V, Zamanian RT, Quertermous T, and Chun HJ. 2011. Disruption of the apelin-APJ system worsens hypoxia-induced pulmonary hypertension. Arterioscler Thromb Vasc Biol 31(4):814-820.
Chaouat A, Coulet F, Favre C, Simonneau G, Weitzenblum E, Soubrier F, et al. Endoglin germline mutation in a patient with hereditary haemorrhagic telangiectasia and dexfenfluramine associated pulmonary arterial hypertension. Thorax. 2004;59(5):446-8.
Chida A, Shintani M, Nakayama T, Furutani Y, Hayama E, Inai K, et al. Missense mutations of the BMPR1B (ALK6) gene in childhood idiopathic pulmonary arterial hypertension. Circ J. 2012;76(6):1501-8.
Clipson A, Wang M, de Leval L, Ashton-Key M, Wotherspoon A, Vassiliou G, Bolli N, Grove C, Moody S, Escudero-Ibarz L et al.. 2015. KLF2 mutation is the most frequent somatic change in splenic marginal zone lymphoma and identifies a subset with distinct genotype. Leukemia 29(5):1177-1185.
Dekker RJ, van Thienen JV, Rohlena J, de Jager SC, Elderkamp YW, Seppen J, de Vries CJ, Biessen EA, van Berkel TJ, Pannekoek H et al.. 2005. Endothelial KLF2 links local arterial shear stress levels to the expression of vascular tone-regulating genes. Am J Pathol 167(2):609-618.
Deng Z, Morse JH, Slager SL, Cuervo N, Moore KJ, Venetos G, et al. Familial primary pulmonary hypertension (gene PPH1) is caused by mutations in the bone morphogenetic protein receptor-II gene. Am J Hum Genet. 2000;67(3):737-44.
de Jesus Perez VA, Yuan K, Lyuksyutova MA, Dewey F, Orcholski ME, Shuffle EM, et al. Whole-Exome Sequencing Reveals TopBP1 as a Novel Gene in Idiopathic Pulmonary Arterial Hypertension. Am J Respir Crit Care Med. 2014;189(10):1260-72.
den Dunnen JT, Dalgleish R, Maglott DR, Hart RK, Greenblatt MS, McGowan-Jordan J, Roux AF, Smith T, Antonarakis SE, Taschner PE et al.. 2016. HGVS Recommendations for the Description of Sequence Variants: 2016 Update. Hum Mutat 37(6):564-569.
Dungey A, Deng Y, Yin J, Rozowsky S, and Stewart DJ. 2011. Abstract 17982: Krüppel-Like Transcription Factor-2 Preserves Endothelial Function and Protects Against Pulmonary Hypertension. Circulation 124(Suppl 21):A17982.
Ehlken N, Lichtblau M, Klose H, Weidenhammer J, Fischer C, Nechwatal R, Uiker S, Halank M, Olsson K, Seeger W et al..2016. Exercise training improves peak oxygen consumption and haemodynamics in patients with severe pulmonary arterial hypertension and inoperable chronic thrombo-embolic pulmonary hypertension: a prospective, randomized, controlled trial. Eur Heart J 37(1):35-44.
Eichstaedt CA, Song J, Benjamin N, Harutyunova S, Fischer C, Grünig E, and Hinderhofer K. EIF2AK4 mutation as "second hit" in hereditary pulmonary arterial hypertension. Respir Res. 2016;17(1):141.
Eichstaedt CA, Song J, Rodriguez Viales R, Pan Z, Benjamin N, Fischer C, Hoeper M, Ulrich S, Hinderhofer K, and Grünig E. First identification of Krüppel-like factor 2 mutation in heritable pulmonary arterial hypertension. Clin Sci. 2017;131(8):689-698.
Evans JD, Girerd B, Montani D, Wang XJ, Galie N, Austin ED, et al. BMPR2 mutations and survival in pulmonary arterial hypertension: an individual participant data meta-analysis. Lancet Respir Med. 2016;4(2):129-37.
Eyries M, Montani D, Girerd B, Perret C, Leroy A, Lonjou C, et al. EIF2AK4 mutations cause pulmonary veno-occlusive disease, a recessive form of pulmonary hypertension. Nat Genet. 2014;46(1):65-9.
Forbes SA, Beare D, Gunasekaran P, Leung K, Bindal N, Boutselakis H, Ding M, Bamford S, Cole C, Ward S et al.. 2015. COSMIC: exploring the world's knowledge of somatic mutations in human cancer. Nucleic Acids Res 43(Database issue):D805-811.
Frydman N, Steffann J, Girerd B, Frydman R, Munnich A, Simonneau G, et al. Pre-implantation genetic diagnosis in pulmonary arterial hypertension due to BMPR2 mutation. Eur Respir J. 2012;39(6):1534-5.
Galiè N, Humbert M, Vachiery JL, Gibbs S, Lang I, Torbicki A, et al. 2015 ESC/ERS Guidelines for the diagnosis and treatment of pulmonary hypertension: The Joint Task Force for the Diagnosis and Treatment of Pulmonary Hypertension of the European Society of Cardiology (ESC) and the European Respiratory Society (ERS): Endorsed by: Association for European Paediatric and Congenital Cardiology (AEPC), International Society for Heart and Lung Transplantation (ISHLT). Eur Heart J. 2016;37(1):67-119.
Girerd B, Montani D, Coulet F, Sztrymf B, Yaici A, Jais X, Tregouet D, Reis A, Drouin-Garraud V, Fraisse A et al.. 2010. Clinical outcomes of pulmonary arterial hypertension in patients carrying an ACVRL1 (ALK1) mutation. Am J Respir Crit Care Med 181:851-861.
Girerd B, Coulet F, Jais X, Eyries M, Van Der Bruggen C, De Man F, et al. Characteristics of pulmonary arterial hypertension in affected carriers of a mutation located in the cytoplasmic tail of bone morphogenetic protein receptor type 2. Chest. 2015;147(5):1385-94.
Girerd B, Montani D, Jais X, Eyries M, Yaici A, Sztrymf B, et al. Genetic counselling in a national referral centre for pulmonary hypertension. Eur Respir J. 2016;47(2):541-52.
Gómez J, Reguero JR, Alvarez C, Junquera MR, Arango A, Morís C, Coto E. A Semiconductor Chip-Based Next Generation Sequencing Procedure for the Main Pulmonary Hypertension Genes. Lung. 2015;193(4):571-4.
Granzow M, Paramasivam N, Hinderhofer K, Fischer C, Chotewutmontri S, Kaufmann L, et al. Loss of function of PGAP1 as a cause of severe encephalopathy identified by Whole Exome Sequencing: Lessons of the bioinformatics pipeline. Mol Cell Probes. 2015;29(5):323-9.
Grünig E, Janssen B, Mereles D, Barth U, Borst MM, Vogt IR, et al. Abnormal pulmonary artery pressure response in asymptomatic carriers of primary pulmonary hypertension gene. Circulation. 2000;102(10):1145-50.
Grünig E, Weissmann S, Ehlken N, Fijalkowska A, Fischer C, Fourme T, et al. Stress Doppler echocardiography in relatives of patients with idiopathic and familial pulmonary arterial hypertension: results of a multicenter European analysis of pulmonary artery pressure response to exercise and hypoxia. Circulation. 2009;119(13):1747-57.
Grünig E, Barner A, Bell M, Claussen M, Dandel M, Dumitrescu D, et al. Non-invasive diagnosis of pulmonary hypertension: ESC/ERS Guidelines with Updated Commentary of the Cologne Consensus Conference 2011. Int J Cardiol. 2011;154 Suppl 1:S3-12.
Harrison RE, Flanagan JA, Sankelo M, Abdalla SA, Rowell J, Machado RD, et al. Molecular and functional analysis identifies ALK-1 as the predominant cause of pulmonary hypertension related to hereditary haemorrhagic telangiectasia. J Med Genet. 2003;40(12):865-71.
He H, Singh I, Wek SA, Dey S, Baird TD, Wek RC, and Georgiadis MM. 2014. Crystal structures of GCN2 protein kinase C-terminal domains suggest regulatory differences in yeast and mammals. J Biol Chem 289(21):15023-15034.
Hinderhofer K, Fischer C, Pfarr N, Szamalek-Hoegel J, Lichtblau M, Nagel C, Egenlauf B, Ehlken N, and Grünig E. 2014. Identification of a new intronic BMPR2-mutation and early diagnosis of heritable pulmonary arterial hypertension in a large family with mean clinical follow-up of 12 years. PLoS One 9(3):e91374.
Kabata H, Satoh T, Kataoka M, Tamura Y, Ono T, Yamamoto M, et al. Bone morphogenetic protein receptor type 2 mutations, clinical phenotypes and outcomes of Japanese patients with sporadic or familial pulmonary hypertension. Respirology. 2013;18(7):1076-82.
Kabitz HJ, Schwoerer A, Bremer HC, Sonntag F, Walterspacher S, Walker D, et al. Impairment of respiratory muscle function in pulmonary hypertension. Clin Sci (Lond). 2008;114(2):165-71.
Kim J. 2014. Apelin-APJ signaling: a potential therapeutic target for pulmonary arterial hypertension. Mol Cells 37(3):196-201.
Kumar A, Lin Z, SenBanerjee S, and Jain MK. 2005. Tumor necrosis factor alpha-mediated reduction of KLF2 is due to inhibition of MEF2 by NF-kappaB and histone deacetylases. Mol Cell Biol 25(14):5893-5903.
Kwapiszewska G, Rodriguez Viales R, Ehlken N, Eichstaedt C, Riemkasten G, Grünig G, et al. Epigenetik und Genetik der pulmonal arteriellen Hypertonie - neue Erkenntnisse der letzten Jahre. Dtsch Med Wochenschr. 2014;139(S 04):111-5.
Lane KB, Machado RD, Pauciulo MW, Thomson JR, Phillips JA, 3rd, Loyd JE, et al. Heterozygous germline mutations in BMPR2, encoding a TGF-beta receptor, cause familial primary pulmonary hypertension. Nat Genet. 2000;26(1):81-4.
Larkin EK, Newman JH, Austin ED, Hemnes AR, Wheeler L, Robbins IM, et al. Longitudinal analysis casts doubt on the presence of genetic anticipation in heritable pulmonary arterial hypertension. Am J Respir Crit Care Med. 2012;186(9):892-6.
Lee JS, Yu Q, Shin JT, Sebzda E, Bertozzi C, Chen M, Mericko P, Stadtfeld M, Zhou D, Cheng L et al.. 2006. Klf2 is an essential regulator of vascular hemodynamic forces in vivo. Dev Cell 11(6):845-857.
Lek M, Karczewski KJ, Minikel EV, Samocha KE, Banks E, Fennell T, O'Donnell-Luria AH, Ware JS, Hill AJ, Cummings BB, et al. Analysis of protein-coding genetic variation in 60,706 humans. Nature. 2016;536(7616):285-291.
Levy M, Eyries M, Szezepanski I, Ladouceur M, Nadaud S, Bonnet D, et al. Genetic analyses in a cohort of children with pulmonary hypertension. Eur Respir J. 2016;48(4):1118-26.
Lyle MA, Fenstad ER, McGoon MD, Frantz RP, Krowka MJ, Kane GC, and Swanson KL. Pulmonary Hypertension in Hereditary Hemorrhagic Telangiectasia. Chest. 2016;149(2):362-371.
Machado RD, Southgate L, Eichstaedt CA, Aldred MA, Austin ED, Best DH, et al. Pulmonary Arterial Hypertension: A Current Perspective on Established and Emerging Molecular Genetic Defects. Hum Mutat. 2015;36(12):1113-27.
Miller SA, Dykes DD, and Polesky HF. 1988. A simple salting out procedure for extracting DNA from human nucleated cells. Nucleic Acids Res 16(3):1215.
Montani D, Achouh L, Dorfmüller P, Le Pavec J, Sztrymf B, Tcherakian C, Rabiller A, Haque R, Sitbon O, Jais X et al.. 2008. Pulmonary veno-occlusive disease: clinical, functional, radiologic, and hemodynamic characteristics and outcome of 24 cases confirmed by histology. Medicine (Baltimore) 87(4):220-233.
Montani D, Lau EM, Dorfmuller P, Girerd B, Jais X, Savale L, Perros F, Nossent E, Garcia G, Parent F et al.. 2016. Pulmonary veno-occlusive disease. Eur Respir J 47(5):1518-1534.
Nagel C, Henn P, Ehlken N, D'Andrea A, Blank N, Bossone E, Bottger A, Fiehn C, Fischer C, Lorenz HM et al.. 2015. Stress Doppler echocardiography for early detection of systemic sclerosis-associated pulmonary arterial hypertension. Arthritis Res Ther 17:165.
Narasimhan J, Staschke KA, and Wek RC. 2004. Dimerization is required for activation of eIF2 kinase Gcn2 in response to diverse environmental stress conditions. J Biol Chem 279(22):22820-22832.
Nasim MT, Ghouri A, Patel B, James V, Rudarakanchana N, Morrell NW, et al. Stoichiometric imbalance in the receptor complex contributes to dysfunctional BMPR-II mediated signalling in pulmonary arterial hypertension. Hum Mol Genet. 2008;17(11):1683-94.
Newman JH, Wheeler L, Lane KB, Loyd E, Gaddipati R, Phillips JA, 3rd, et al. Mutation in the gene for bone morphogenetic protein receptor II as a cause of primary pulmonary hypertension in a large kindred. N Engl J Med. 2001;345(5):319-24.
Padyana AK, Qiu H, Roll-Mecak A, Hinnebusch AG, and Burley SK. 2005. Structural basis for autoinhibition and mutational activation of eukaryotic initiation factor 2alpha protein kinase GCN2. J Biol Chem 280(32):29289-29299.
Pfarr N, Szamalek-Hoegel J, Fischer C, Hinderhofer K, Nagel C, Ehlken N, et al. Hemodynamic and clinical onset in patients with hereditary pulmonary arterial hypertension and BMPR2 mutations. Respir Res. 2011;12:99.
Piao C, Zhu Y, Zhang C, Xi X, Liu X, Zheng S, Li X, Guo J, Jia L, Nakanishi T, Cai T, Gu H, Du J. Identification of multiple ACVRL1 mutations in patients with pulmonary arterial hypertension by targeted exome capture. Clin Sci (Lond). 2016;130(17):1559-69.
Piva R, Deaglio S, Fama R, Buonincontri R, Scarfo I, Bruscaggin A, Mereu E, Serra S, Spina V, Brusa D et al.. 2015. The Krüppel-like factor 2 transcription factor gene is recurrently mutated in splenic marginal zone lymphoma. Leukemia 29(2):503-507.
Remillard CV, Tigno DD, Platoshyn O, Burg ED, Brevnova EE, Conger D, et al. Function of Kv1.5 channels and genetic variations of KCNA5 in patients with idiopathic pulmonary arterial hypertension. Am J Physiol Cell Physiol. 2007;292(5):C1837-53.
Richards S, Aziz N, Bale S, Bick D, Das S, Gastier-Foster J, et al. Standards and guidelines for the interpretation of sequence variants: a joint consensus recommendation of the American College of Medical Genetics and Genomics and the Association for Molecular Pathology. Genet Med. 2015;17(5):405-24.
Rodriguez Viales R, Eichstaedt CA, Ehlken N, Fischer C, Lichtblau M, Grünig E, et al. Mutation in BMPR2 Promoter: A 'Second Hit' for Manifestation of Pulmonary Arterial Hypertension? PLoS One. 2015;10(7):e0133042.
Schroeder C, Faust U, Sturm M, Hackmann K, Grundmann K, Harmuth F, et al. HBOC multi-gene panel testing: comparison of two sequencing centers. Breast Cancer Res Treat. 2015;152(1):129-36.
SenBanerjee S, Lin Z, Atkins GB, Greif DM, Rao RM, Kumar A, Feinberg MW, Chen Z, Simon DI, Luscinskas FW et al.. 2004. KLF2 Is a novel transcriptional regulator of endothelial proinflammatory activation. J Exp Med 199(10):1305-1315.
Shintani M, Yagi H, Nakayama T, Saji T, Matsuoka R. A new nonsense mutation of SMAD8 associated with pulmonary arterial hypertension. J Med Genet. 2009;46(5):331-7.
Song J, Eichstaedt CA, Rodriguez Viales R, Benjamin N, Harutyunova S, Fischer C, Grünig E, and Hinderhofer K. 2016. Identification of genetic defects in pulmonary arterial hypertension by a new panel diagnostic. Clin Sci (Lond) 22:2043-2052.
Soubrier F, Chung WK, Machado R, Grünig E, Aldred M, Geraci M, et al. Genetics and genomics of pulmonary arterial hypertension. J Am Coll Cardiol. 2013;62(25 Suppl):D13-21.
Sztrymf B, Yaici A, Girerd B, Humbert M. Genes and pulmonary arterial hypertension. Respiration. 2007;74(2):123-32.
Tenorio J, Navas P, Barrios E, Fernandez L, Nevado J, Quezada CA, et al. A founder EIF2AK4 mutation causes an aggressive form of pulmonary arterial hypertension in Iberian Gypsies. Clin Genet. 2014;88(6):579-83.
The 1000 Genomes Project Consortium, Auton A, Brooks LD, Durbin RM, Garrison EP, Kang HM, Korbel JO, Marchini JL, McCarthy S, McVean GA et al.. 2015. A global reference for human genetic variation. Nature 526(7571):68-74.
Torring PM, Brusgaard K, Ousager LB, Andersen PE, and Kjeldsen AD. National mutation study among Danish patients with hereditary haemorrhagic telangiectasia. Clin Genet. 2014;86(2):123-133.
Trembath RC, Thomson JR, Machado RD, Morgan NV, Atkinson C, Winship I, et al. Clinical and molecular genetic features of pulmonary hypertension in patients with hereditary hemorrhagic telangiectasia. N Engl J Med. 2001;345(5):325-34.
Wallis Y, Payne S, McAnulty C, Bodmer D, Sistermans E, Robertson K, et al. Practice Guidelines for the Evaluation of Pathogenicity and the Reporting of Sequence Variants in Clinical Molecular Genetics. ACGS/ VKGL. 2013.
Wang G, Knight L, Ji R, Lawrence P, Kanaan U, Li L, et al. Early onset severe pulmonary arterial hypertension with 'two-hit' digenic mutations in both BMPR2 and KCNA5 genes. Int J Cardiol. 2014;177(3):e167-9.
Xi Q, Liu Z, Zhao Z, Luo Q, Huang Z. High Frequency of Pulmonary Hypertension-Causing Gene Mutation in Chinese Patients with Chronic Thromboembolic Pulmonary Hypertension. PLoS One 2016; 11: e0147396.
Yeo JC, Jiang J, Tan ZY, Yim GR, Ng JH, Goke J, Kraus P, Liang H, Gonzales KA, Chong HC et al.. 2014. Klf2 is an essential factor that sustains ground state pluripotency. Cell stem cell 14(6):864-872.

## Claims

1. Use of a gene panel or combination of genes comprising at least the genes with the nucleotide sequence of SEQ ID Nos. 1 to 14 in the in *vitro* or *ex vivo* diagnosis of a genetic disposition to pulmonary arterial hypertension (PAH) and/or chronic thromboembolic pulmonary hypertension (CTEPH), wherein the gene panel or combination of genes comprises the at least 14 genes with the nucleotide sequence of SEQ ID Nos. 1 to 14 and up to the 50 genes comprising genes with the nucleotide sequence of SEQ ID Nos. 1 to 43.

2. The use of claim 1, wherein the gene panel or combination of genes comprises at least the genes with the nucleotide sequence of SEQ ID Nos. 1 to 36, preferably comprises the genes with the nucleotide sequence of SEQ ID Nos. 1 to 43.

3. The use according to any one of claims 1 to 2, wherein the gene panel or combination of genes comprises the at least the genes with the nucleotide sequence of SEQ ID Nos. 1 to 14 and 42.

4. The use according to any one of claims 1 to 3, comprising the in *vitro* or *ex vivo* diagnosis of a genetic disposition to pulmonary veno-occlusive disease (PVOD) and/or hereditary haemorrhagic telangiectasia (HHT).

5. The use according to any one of claims 1 to 4, comprising sequencing of the exons and exon-intron boundaries of the genes and determining whether at least one mutation in terms of one or more altered base pairs, smaller and larger deletions or insertions in the genes is present,
preferably comprising the use of next generation sequencing,
optionally further comprising an additional sequencing, preferably Sanger sequencing,
or an analysis technique, preferably multiplex ligation-dependent probe amplification or quantitative polymerase chain reaction.

6. The use according to any one of claims 1 to 5, comprising simultaneous testing of at least two patient samples,
wherein a patient sample is preferably whole blood, peripheral blood or tissue.

7. The use according to any one of claims 1 to 6, comprising
- diagnostic genetic testing in subjects, preferably PAH/CTEPH/PVOD/HHT patients and/or their family members;
- screening of newborns; and/or
- pre-implantation diagnostics.

8. A method for the diagnosis of genetic defects in pulmonary arterial hypertension (PAH) and/or chronic thromboembolic pulmonary hypertension (CTEPH), comprising the following steps:
(a) extracting the genomic DNA from at least one sample, preferably at least two samples, wherein the sample(s) has/have been obtained from a patient or from a relative of a PAH/CTEPH patient,
(b) determining whether at least one mutation in terms of one or more altered base pairs, smaller and larger deletions or insertions in the genes with the nucleotide sequence of SEQ ID Nos. 1 to 14 is present by using next generation sequencing,
wherein the presence of said at least one such mutation is a genetic confirmation of existing PAH or indicative for the increased probability for PAH/CTEPH to manifest in not yet affected relatives of PAH/CTEPH patients, and
(c) optionally, determining whether detected mutations/gene variants have pathological consequences either predicted with *in silico* prediction programmes and/or functional studies
wherein in step (b) the presence of at least one mutation in terms of one or more altered base pairs, smaller and larger deletions or insertions in the 14 genes with the nucleotide sequence of SEQ ID Nos. 1 to 14 and in up to 50 genes is determined, wherein said 50 genes comprise the genes with the nucleotide sequence of SEQ ID Nos. 1 to 43.

9. The method of claim 8, wherein in step (b) the presence of at least one mutation in terms of one or more altered base pairs, smaller and larger deletions or insertions in the genes with the nucleotide sequence of SEQ ID Nos. 1 to 36 is determined, preferably in the genes with the nucleotide sequence of SEQ ID Nos. 1 to 43,
and/or wherein in step (b) the presence of at least one mutation in terms of one or more altered base pairs, smaller and larger deletions or insertions in the genes with the nucleotide sequence of SEQ ID Nos. 1 to 14 and 42 is determined.

10. The method of claim 8 or 9, wherein the diagnosis of genetic defects in pulmonary arterial hypertension comprises the diagnosis for pulmonary veno-occlusive disease (PVOD) and/or hereditary haemorrhagic telangiectasia (HHT).

11. The method of any one of claim 8 to 10, wherein the presence of at least two mutations is indicative for the existence of PAH/CTEPH/PVOD/HHT or the increased probability for PAH/CTEPH/PVOD/HHT to become manifest,
and/or wherein the (patient) sample is whole blood, peripheral blood or tissue,
and/or wherein at least two, preferably up to about 96 (patient) samples are tested simultaneously,
and/or wherein the method is used for
- diagnostic genetic testing in subjects, preferably PAH/CTEPH/PVOD/HHT patients and/or its family members;
- screening of newborns; and/or
- pre-implantation diagnostics.

12. The method of any one of claims 8 to 11, comprising the further step of
(d) carrying out an additional sequencing,
preferably Sanger sequencing,
or an analysis technique,
preferably multiplex ligation-dependent probe amplification or quantitative polymerase chain reaction.

13. Kit for the diagnosis of genetic defects in pulmonary arterial hypertension (PAH) and/or chronic thromboembolic pulmonary hypertension (CTEPH), comprising
(i) means for carrying out next generation sequencing and detecting at least one mutation in terms of one or more altered base pairs, smaller and larger deletions or insertions in the genes with the nucleotide sequence of SEQ ID Nos. 1 to 14, wherein said means comprise probes suitable for sequencing target genomic regions of the respective genes and for determining whether at least one such mutation in said genes is present,
(ii) optionally, means for processing genomic DNA,
(iii) optionally, means for carrying out an additional sequencing, preferably Sanger sequencing, or an analysis technique, preferably multiplex ligation-dependent probe amplification or quantitative polymerase chain reaction.

14. The kit of claim 13, comprising means for carrying out next generation sequencing and detecting at least one mutation in terms of one or more altered base pairs, smaller and larger deletions or insertions in the genes with the nucleotide sequence of SEQ ID Nos. 1 to 36, preferably in the genes with the nucleotide sequence of SEQ ID Nos. 1 to 43,
and/or comprising means for carrying out next generation sequencing and detecting at least one mutation in terms of one or more altered base pairs, smaller and larger deletions or insertions in the genes with the nucleotide sequence of SEQ ID Nos. 1 to 14 and 42.

## Patentansprüche

1. Verwendung eines Gen-Panels oder einer Kombination von Genen umfassend mindestens die Gene mit der Nukleotidsequenz von SEQ ID Nos. 1 bis 14 in der *in vitro* oder *ex vivo* Diagnose einer genetischen Disposition für pulmonale arterielle Hypertonie (PAH) und/oder chronische thromboembolische pulmonale Hypertonie (CTEPH), wobei das Gen-Panel oder die Kombination von Genen die mindestens 14 Gene mit der Nukleotidsequenz von SEQ ID Nos. 1 bis 14 und bis zu den 50 Genen umfassend Gene mit der Nukleotidsequenz von SEQ ID Nos. 1 bis 43 umfasst.

2. Verwendung gemäß Anspruch 1, wobei das Gen-Panel oder die Kombination von Genen mindestens Gene mit der Nukleotidsequenz von SEQ ID Nos. 1 bis 36 umfasst, bevorzugt Gene mit der Nukleotidsequenz von SEQ ID Nos. 1 bis 43 umfasst.

3. Verwendung gemäß irgendeinem der Ansprüche 1 bis 2, wobei das Gen-Panel oder die Kombination von Genen mindestens die Gene mit der Nukleotidsequenz von SEQ ID Nos. 1 bis 14 und 42 umfasst.

4. Verwendung gemäß irgendeinem der Ansprüche 1 bis 3, umfassend die in *vitro* oder *ex vivo* Diagnose einer genetischen Disposition für pulmonale veno-okklusive Erkrankung (PVOD) und/oder hereditäre hämorrhagische Telangiektasie (HHT).

5. Verwendung gemäß irgendeinem der Ansprüche 1 bis 4, umfassend Sequenzieren der Exons und Exon-Intron-Grenzen der Gene und Bestimmung, ob mindestens eine Mutation in Bezug auf eines oder mehrere geänderte Basenpaare, kleinere und größere Deletionen oder Insertionen in den Genen vorhanden ist,
bevorzugt umfassend die Verwendung von Next-Generation Sequenzierung,
optional weiter umfassend eine zusätzliche Sequenzierung, bevorzugt Sanger-Sequenzierung, oder eine Analysetechnik, bevorzugt multiplexe ligationsabhängige Sondenamplifikation oder quantitative Polymerase-Kettenreaktion.

6. Verwendung gemäß irgendeinem der Ansprüche 1 bis 5, umfassend simultanes Testen von mindestens 2 Patientenproben,
wobei eine Patientenprobe bevorzugt Vollblut, periphäres Blut oder Gewebe ist.

7. Verwendung gemäß irgendeinem der Ansprüche 1 bis 6, umfassend
- diagnostische genetische Testung in Subjekten, bevorzugt PAH/CTEPH/PVOD/HHT Patienten und/oder deren Familienmitgliedern;
- Screening von Neugeborenen; und/oder
- Präimplantations-Diagnostik.

8. Verfahren zur Diagnose von genetischen Defekten bei pulmonaler arterieller Hypertonie (PAH) und/oder chronischer thromboembolischer pulmonaler Hypertonie (CTEPH), umfassend die folgenden Schritte:
(a) Extrahieren der genomischen DNA aus mindestens einer Probe, bevorzugt mindestens 2 Proben, wobei der/die Probe/n von einem Patienten oder einem Verwandten eines PAH/CTEPH-Patienten erhalten wurde/n,
(b) Bestimmen, ob mindestens eine Mutation in Bezug auf eines oder mehrere geänderte Basenpaare, kleinere und größere Deletionen oder Insertionen in den Genen mit der Nukleotidsequenz von SEQ ID Nos. 1 bis 14 vorhanden ist unter der Verwendung von Next-Generation Sequenzierung,
wobei die Anwesenheit der mindestens einen solchen Mutation eine genetische Bestätigung für das Vorhandensein von PAH oder indikativ für die erhöhte Wahrscheinlichkeit ist, dass sich PAH/CTEPH in bisher noch nicht betroffenen Verwandten von PAH/CTEPH-Patienten manifestieren wird,
und
(c) optional, Bestimmen, ob detektierte Mutationen/Genvarianten pathologische Konsequenzen haben, entweder vorhergesagt mit *in silico* Vorhersageprogrammen und/oder funktionalen Studien,
wobei in Schritt (b) die Anwesenheit der mindestens einen Mutation in Bezug auf eines oder mehrere geänderte Basenpaare, kleinere und größere Deletionen oder Insertionen in den 14 Genen mit der Nukleotidsequenz von SEQ ID Nos. 1 bis 14 in bis zu 50 Genen bestimmt wird, wobei die 50 Gene die Gene mit der Nukleotidsequenz von SEQ ID Nos. 1 bis 43 umfassen.

9. Das Verfahren gemäß Anspruch 8, wobei in Schritt (b) die Anwesenheit der mindestens einen Mutation in Bezug auf eines oder mehrere geänderte Basenpaare, kleinere und größere Deletionen oder Insertionen in den Genen mit der Nukleotidsequenz von SEQ ID Nos. 1 bis 36 bestimmt wird, bevorzugt in den Genen mit der Nukleotidsequenz von SEQ ID Nos. 1 bis 43,
und/oder wobei in Schritt (b) die Anwesenheit der mindestens einen Mutation in Bezug auf eines oder mehrere geänderte Basenpaare, kleinere und größere Deletionen oder Insertionen in den Genen mit der Nukleotidsequenz von SEQ ID Nos. 1 bis 14 und 42 bestimmt wird.

10. Das Verfahren gemäß Anspruch 8 oder 9, wobei die Diagnose von genetischen Defekten bei pulmonaler arterieller Hypertonie die Diagnose für pulmonale veno-okklusive Erkrankung (PVOD) und/oder hereditäre hämorrhagische Telangiektasie (HHT) umfasst.

11. Das Verfahren gemäß irgendeinem der Ansprüche 8 bis 10, wobei die Anwesenheit von mindestens 2 Mutation indikativ für das Vorhandensein von PAH/CTEPH/PVOD/HHT oder für die erhöhte Wahrscheinlichkeit ist, dass sich PAH/CTEPH/PVOD/HHT manifestieren wird,
und/oder wobei die (Patienten-) Probe Vollblut, periphäres Blut oder Gewebe ist,
und/oder wobei mindestens 2, bevorzugt bis zu ungefähr 96 (Patienten-) Proben simultan getestet werden,
und/oder wobei das Verfahren verwendet wird für
- diagnostische genetische Testung in Subjekten, bevorzugt PAH/CTEPH/PVOD/HHT - Patienten und/oder deren Familienmitgliedern;
- Screening von Neugeborenen; und/oder
- Präimplantations-Diagnostik.

12. Das Verfahren gemäß irgendeinem der Ansprüche 8 bis 11, umfassend den weiteren Schritt:
(d) Durchführen einer zusätzlicher Sequenzierung,
bevorzugt Sanger-Sequenzierung,
oder einer Analysetechnik,
bevorzugt multiplexe ligationsabhängige Sondenamplifikation oder quantitative Polymerase-Kettenreaktion.

13. Kit für die Diagnose von genetischen Defekten bei pulmonaler arterieller Hypertonie (PAH) und/oder chronischer thromboembolischer pulmonaler Hypertonie (CTEPH), umfassend
(i) Mittel zum Durchführen von Next-Generation Sequenzierung und Detektieren mindestens einer Mutation in Bezug auf eines oder mehrere geänderte Basenpaare, kleinere und größere Deletionen oder Insertionen in den Genen mit der Nukleotidsequenz von SEQ ID Nos. 1 bis 14, wobei die Mittel Sonden umfassen, die geeignet sind für Sequenzierung von Zielregionen der jeweiligen Gene und für Bestimmung, ob mindestens eine solche Mutation in den Genen vorkommt.
(ii) optional, Mittel für die Verarbeitung von genomischer DNA,
(iii) optional, Mittel zum Durchführen einer zusätzlichen Sequenzierung, bevorzugt bevorzugt Sanger-Sequenzierung oder einer Analysetechnik, bevorzugt multiplexe ligationsabhängige Sondenamplifikation oder quantitative Polymerase-Kettenreaktion.

14. Der Kit gemäß Anspruch 13, umfassend Mittel zum Durchführen von Next-Generation Sequenzierung und Detektieren mindestens einer Mutation in Bezug auf eines oder mehrere geänderte Basenpaare, kleinere und größere Deletionen oder Insertionen in den Genen mit der Nukleotidsequenz von 1 bis 36, bevorzugt in den Genen mit der Nukleotidsequenz von SEQ ID Nos. 1 bis 43,
und/oder umfassend Mittel zum Durchführen von Next-Generation Sequenzierung und Detektieren mindestens einer Mutation in Bezug auf eines oder mehrere geänderte Basenpaare, kleinere und größere Deletionen oder Insertionen in den Genen mit der Nukleotidsequenz von 1 bis 14 und 42.

## Revendications

1. Utilisation d'un panel de gènes ou d'une combinaison de gènes comprenant au moins les gènes possédant la séquence de nucléotides de SEQ ID NO: 1 à 14 dans le diagnostic *in vitro* ou *ex vivo* d'une disposition génétique à l'hypertension artérielle pulmonaire (HTAP) et/ou l'hypertension pulmonaire thromboembolique chronique (HTP-TEC), dans laquelle le panel de gènes ou la combinaison de gènes comprend les au moins 14 gènes possédant la séquence de nucléotides de SEQ ID NO: 1 à 14 et jusqu'à les 50 gènes comprenant les gènes possédant la séquence de nucléotides de SEQ ID NO: 1 à 43.

2. Utilisation selon la revendication 1, dans laquelle le panel de gènes ou la combinaison de gènes comprend au moins les gènes possédant la séquence de nucléotides de SEQ ID NO: 1 à 36, de préférence comprend les gènes possédant la séquence de nucléotides de SEQ ID NO: 1 à 43.

3. Utilisation selon l'une quelconque des revendications 1 à 2, dans laquelle le panel de gènes ou la combinaison de gènes comprend les au moins les gènes possédant la séquence de nucléotides de SEQ ID NO: 1 à 14 et 42.

4. Utilisation selon l'une quelconque des revendications 1 à 3, comprenant le diagnostic *in vitro* ou *ex vivo* d'une disposition génétique à la maladie veino-occlusive pulmonaire (MVOP) et/ou la télangiectasie hémorragique héréditaire (THH).

5. Utilisation selon l'une quelconque des revendications 1 à 4, comprenant le séquençage des exons et des frontières exon-intron des gènes et le fait de déterminer si au moins une mutation, en termes d'une ou plusieurs paires de bases altérées, de délétions ou d'insertions plus petites et plus grandes dans les gènes, est présente,
de préférence comprenant l'utilisation d'un séquençage de nouvelle génération, facultativement comprenant en outre un séquençage supplémentaire, de préférence un séquençage de Sanger, ou une technique d'analyse, de préférence une amplification multiplex de sondes dépendant d'une ligation ou une réaction en chaîne par polymérase quantitative.

6. Utilisation selon l'une quelconque des revendications 1 à 5, comprenant le test simultané d'au moins deux échantillons de patient,
dans laquelle un échantillon de patient est de préférence du sang total, du sang périphérique ou un tissu.

7. Utilisation selon l'une quelconque des revendications 1 à 6, comprenant
- un test génétique diagnostique chez des sujets, de préférence des patients souffrant de HTAP/HTP-TEC/MVOP/THH et/ou les membres de leur famille ;
- le dépistage de nouveau-nés ; et/ou
- un diagnostic pré-implantation.

8. Procédé pour le diagnostic d'anomalies génétiques dans l'hypertension artérielle pulmonaire (HTAP) et/ou l'hypertension pulmonaire thromboembolique chronique (HTP-TEC), comprenant les étapes suivantes consistant à :
(a) extraire l'ADN génomique d'au moins un échantillon, de préférence d'au moins deux échantillons, où l'échantillon/les échantillons a/ont été obtenu(s) chez un patient ou chez un proche d'un patient souffrant de HTAP/HTP-TEC,
(b) déterminer si au moins une mutation, en termes d'une ou plusieurs paires de bases altérées, de délétions ou d'insertions plus petites et plus grandes dans les gènes possédant la séquence de nucléotides de SEQ ID NO: 1 à 14, est présente en utilisant un séquençage de nouvelle génération,
dans lequel la présence de ladite au moins une telle mutation est une confirmation génétique d'une HTAP existante ou est indicative de la probabilité accrue qu'une HTAP/HTP-TEC se manifeste chez des proches, non encore affectés, des patients souffrant de HTAP/HTP-TEC, et
(c) facultativement, déterminer si les mutations/variants génétiques détecté(e)s ont des conséquences pathologiques, prédites par des programmes de prédiction *in silico* et/ou des études fonctionnelles
dans lequel, à l'étape (b), la présence d'au moins une mutation, en termes d'une ou plusieurs paires de bases altérées, de délétions ou d'insertions plus petites et plus grandes dans les 14 gènes possédant la séquence de nucléotides de SEQ ID NO: 1 à 14 et dans jusqu'à 50 gènes, est déterminée, dans lequel lesdits 50 gènes comprennent les gènes possédant la séquence de nucléotides de SEQ ID NO: 1 à 43.

9. Procédé selon la revendication 8, dans lequel, à l'étape (b), la présence d'au moins une mutation, en termes d'une ou plusieurs paires de bases altérées, de délétions ou d'insertions plus petites et plus grandes dans les gènes possédant la séquence de nucléotides de SEQ ID NO: 1 à 36, est déterminée, de préférence dans les gènes possédant la séquence de nucléotides de SEQ ID NO: 1 à 43,
et/ou dans lequel, à l'étape (b), la présence d'au moins une mutation, en termes d'une ou plusieurs paires de bases altérées, de délétions ou d'insertions plus petites et plus grandes dans les gènes possédant la séquence de nucléotides de SEQ ID NO: 1 à 14 et 42, est déterminée.

10. Procédé selon la revendication 8 ou 9, dans lequel le diagnostic d'anomalies génétiques dans l'hypertension artérielle pulmonaire comprend le diagnostic de la maladie veino-occlusive pulmonaire (MVOP) et/ou la télangiectasie hémorragique héréditaire (THH).

11. Procédé selon l'une quelconque des revendications 8 à 10, dans lequel la présence d'au moins deux mutations est indicative de l'existence d'une HTAP/HTP-TEC/MVOP/THH ou de la probabilité accrue qu'une HTAP/HTP-TEC/MVOP/THH devienne manifeste,
et/ou dans lequel l'échantillon (de patient) est du sang total, du sang périphérique ou un tissu,
et/ou dans lequel au moins deux, de préférence jusqu'à environ 96 échantillons (de patient) sont testés simultanément,
et/ou dans lequel le procédé est utilisé pour
- un test génétique diagnostique chez des sujets, de préférence des patients souffrant de HTAP/HTP-TEC/MVOP/THH et/ou les membres de leur famille ;
- le dépistage de nouveau-nés ; et/ou
- un diagnostic pré-implantation.

12. Procédé selon l'une quelconque des revendications 8 à 11, comprenant l'étape supplémentaire consistant à
(d) réaliser un séquençage supplémentaire,
de préférence un séquençage de Sanger,
ou une technique d'analyse,
de préférence une amplification multiplex de sondes dépendant d'une ligation ou une réaction en chaîne par polymérase quantitative.

13. Kit pour le diagnostic d'anomalies génétiques dans l'hypertension artérielle pulmonaire (HTAP) et/ou l'hypertension pulmonaire thromboembolique chronique (HTP-TEC), comprenant
(i) un moyen pour réaliser un séquençage de nouvelle génération et détecter au moins une mutation, en termes d'une ou plusieurs paires de bases altérées, de délétions ou d'insertions plus petites et plus grandes dans les gènes possédant la séquence de nucléotides de SEQ ID NO: 1 à 14, dans lequel ledit moyen comprend des sondes appropriées pour séquencer des régions génomiques cibles des gènes respectifs et pour déterminer si au moins une telle mutation dans lesdits gènes est présente,
(ii) facultativement, un moyen pour traiter de l'ADN génomique,
(iii) facultativement, un moyen pour réaliser un séquençage supplémentaire, de préférence un séquençage de Sanger, ou une technique d'analyse, de préférence une amplification multiplex de sondes dépendant d'une ligation ou une réaction en chaîne par polymérase quantitative.

14. Kit selon la revendication 13, comprenant un moyen pour réaliser un séquençage de nouvelle génération et détecter au moins une mutation, en termes d'une ou plusieurs paires de bases altérées, de délétions ou d'insertions plus petites et plus grandes dans les gènes possédant la séquence de nucléotides de SEQ ID NO: 1 à 36, de préférence dans les gènes possédant la séquence de nucléotides de SEQ ID NO: 1 à 43,
et/ou comprenant un moyen pour réaliser un séquençage de nouvelle génération et détecter au moins une mutation, en termes d'une ou plusieurs paires de bases altérées, de délétions ou d'insertions plus petites et plus grandes dans les gènes possédant la séquence de nucléotides de SEQ ID NO: 1 à 14 et 42.
